# EUROPÄISCHE PATENTSCHRIFT

(11) **EP 2 421 585 B1**
(45) Veröffentlichungstag und Bekanntmachung des Hinweises auf die Patenterteilung: **29.06.2016**
(21) Anmeldenummer: 10717534.1
(22) Anmeldetag: 15.04.2010
(51) Int. Cl.: A61M 1/36, A61M 39/10

(54) **VERBINDUNGSEINRICHTUNG UND VERFAHREN ZUM KONNEKTIEREN WENIGSTENS ZWEIER FLUIDFÜHRENDER MEDIZINTECHNISCHER SYSTEME, SOWIE MEDIZINTECHNISCHE VORRICHTUNG**
CONNECTING DEVICE AND METHOD FOR CONNECTING AT LEAST TWO FLUID-CONDUCTING MEDICAL SYSTEMS, AS WELL AS MEDICAL-TECHNICAL DEVICE
DISPOSITIF DE RACCORD ET PROCÉDÉ DE RACCORDEMENT D'AU MOINS DEUX SYSTÈMES MÉDICAUX CONDUCTEURS DE FLUIDE, AINSI QUE DISPOSITIF MÉDICAL

(30) Priorität: 23.04.2009 DE 102009018664; 10.06.2009 DE 102009024575; 10.06.2009 US 185687 P
(43) Veröffentlichungstag der Anmeldung: 29.02.2012
(73) Patentinhaber: Fresenius Medical Care Deutschland GmbH, 61352 Bad Homburg (DE)
(72) Erfinder: LAUER, Martin, 66606 St. Wendel (DE)
(74) Vertreter: Bobbert & Partner Patentanwälte PartmbB
(86) Internationale Anmeldenummer: PCT/EP2010/002323
(87) Internationale Veröffentlichungsnummer: WO 2010/121751

(56) Entgegenhaltungen:
- EP-A1- 1 566 196
- EP-A1- 1 649 890
- WO-A1-03/101510
- WO-A1-2006/037638
- US-A- 5 807 347

## Beschreibung

Die vorliegende Erfindung betrifft eine Verbindungseinrichtung gemäß Anspruch 1. Sie betrifft ferner ein Verfahren zum Konnektieren wenigstens zweier fluidführender medizintechnischer Systeme gemäß Anspruch 12 sowie eine externe medizintechnische Funktionseinrichtung gemäß Anspruch 14 und eine medizintechnische Vorrichtung gemäß Anspruch 15.

Bei Behandlungs- und Analysevorrichtungen aus Medizin- oder Labortechnik kommen regelmäßig externe Funktionseinrichtungen zum Einsatz. Solche externen Funktionseinrichtungen, welche auf Schlauchsystemen und Kassettenanordnungen basieren können, werden häufig unter Herstellen einer Fluidverbindung für Flüssigkeiten und/oder Gase mit der Behandlungs- und Analysevorrichtung und/oder einer weiteren externen Funktionseinrichtungen verbunden.

Die EP 1 649 890 A1 offenbart ein Verbindungssystem für den medizinischen Gebrauch.

Die EP 1 566 196 A1 (Nächtliegender Stand der Technik). offenbart eine Verbindungsvorrichtung.

Die WO 2006/037638 offenbart eine Verbindungsstruktur für eine Transfusionsleitung.

Die US 5,807,347 offenbart ein medizinisches Ventilelement.

Die WO 03/101510 A1 offenbart eine Vorrichtung zur Behandlung einer medizinischen Flüssigkeit.

Aufgabe der vorliegenden Erfindung ist, eine weitere Verbindungseinrichtung zur Fluidverbindung zwischen wenigstens zwei fluidführenden, medizinischen Systemen (im Folgenden auch kurz: System oder Systeme) bereitzustellen.

Diese Aufgabe wird durch eine Verbindungseinrichtung mit den Merkmalen des Anspruchs 1 gelöst.

Bei allen folgenden Ausführungen ist der Gebrauch des Ausdrucks "kann sein oder haben" synonym zu "ist oder hat vorzugsweise" zu verstehen.

Die erfindungsgemäße Verbindungseinrichtung dient zur Fluidverbindung von wenigstens einem ersten fluidführenden System und einem zweiten fluidführenden System (unten stehend auch als Konnektionssystem bezeichnet) und weist wenigstens ein erstes Rohrstück des ersten Systems auf, welches vorgesehen ist, mit einem zweiten Rohrstück des zweiten Systems verbunden zu werden.

Mittels der Verbindung der beiden Rohrstücke kann eine Fluidverbindung vorteilhaft zwischen beiden Systemen hergestellt werden. Bei den Systemen kann es sich jeweils um eine externe Funktionseinrichtung und/oder eine Behandlungsvorrichtung handeln, zwischen welchen eine Fluidverbindung hergestellt werden soll.

Der Begriff "Rohr" bzw. "Rohrstück", wie er hierin verwendet wird, bezeichnet einen Rohrkanal oder eine Rohrleitung, welche(r) dazu geeignet und vorgesehen ist, Fluide aufzunehmen und/oder weiterzuleiten.

Vorteilhafte Weiterentwicklungen der vorliegenden Erfindung sind jeweils Gegenstand der Unteransprüche.

So weist in einer bevorzugten erfindungsgemäßen Ausführungsform das erste oder das zweite Rohr bzw. Rohrstück wenigstens ein Innenrohr und wenigstens ein Außenrohr auf. Das Innenrohr ist in einem Inneren des Außenrohrs angeordnet. Das Innenrohr und das Außenrohr können konzentrisch oder nicht konzentrisch zueinander angeordnet sein.

Das Innenrohr und das Außenrohr können an einer externen Funktionseinrichtung oder an einer Behandlungsvorrichtung angeordnet oder zu ihrer Anordnung dort vorgesehen sein.

Die Verbindungseinrichtung kann zum Aufnehmen wenigstens eines Abschnitts des Konnektionsrohrs einer Behandlungsvorrichtung oder einer externen Funktionseinrichtung zwischen dem Innenrohr und dem Außenrohr geeignet sein.

Der Begriff "Konnektionsrohr" bezeichnet ein Rohr, ein Rohrstück, einen Kanal, eine Leitung oder dergleichen, welche mit der erfindungsgemäßen Verbindungseinrichtung konnektierbar bzw. verbindbar oder konnektiert bzw. verbunden ist.

Das Konnektionsrohr kann in jeder erfindungsgemäßen Ausführungsform flexibel oder starr sein.

Das Konnektionsrohr kann mit einem Abschnitt hiervon in einen Zwischenraum zwischen dem Irmenrohr und dem Außenrohr der Verbindungseinrichtung aufgenommen werden.

Zwischen einem Inneren des Innenrohrs und einem Inneren des Außenrohrs kann eine Fluidverbindung vorgesehen sein.

Das erste und/oder das zweite System können jeweils eine externe, medizintechnische Funktionseinrichtung, eine medizintechnische Behandlungs- oder eine Blutbehandlungsvorrichtung, eine medizinische Analysevorrichtung oder dergleichen medizinische Fluide (insbesondere Flüssigkeiten und Gase) führende Systeme sein.

Die Verbindungseinrichtung kann eine Verschlusseinrichtung aufweisen zum Kurzschluss zwischen dem Innenlumen und dem ringförmigen Spalt zwischen dem Innenrohr und dem Außenrohr mit dem Zweck des Spülens und/oder Desinfizieren sämtlicher fluidberührender Innenflächen des Systems.

Die Verbindungseinrichtung kann eine Aufweitung zum Verbinden, insbesondere Verklemmen oder Aufweiten, des Innenrohrs gegen das Konnektionsrohr aufweisen. Die Aufweitung kann am Innenrohr oder am Konnektionsrohr vorliegen. Sie kann einstückig mit dem Innenrohr oder dem Konnektionsrohr hergestellt vorliegen, oder mit diesem verbunden worden sein. Sie kann eine kugelförmige oder konvexe Außenoberfläche aufweisen. Sie kann vorgesehen sein, um im gebrauchsfertigen Verbindungszustand der Verbindungseinrichtung eine Fluidabdichtung zwischen Innenrohr und Konnektionsrohr zu bewirken.

Die Aufweitung kann eine kugelförmige und/oder sphärische und/oder gekrümmte und/oder bogenförmige und/oder konvexe Außenoberfläche, insbesondere in einem Bereich des maximalen Querschnitts oder Durchmessers, vorzugsweise in Strömungsrichtung, aufweisen.

Die Aufweitung kann ausgestaltet sein, um im gebrauchsfertigen Verbindungszustand der Verbindungseinrichtung eine Fluidabdichtung zwischen dem erstem Rohrstück und dem zweitem Rohrstück, insbesondere zwischen Innenrohr und Konnektionsrohr, zu bewirken.

Die Aufweitung kann ihren größten Querschnittsdurchmesser oder ihren größten Umfang in einem Querschnitt in einer Ebene senkrecht zu der Richtung des Verbindens des ersten Rohrstücks mit dem zweiten Rohrstück in einem in Verbindungsrichtung mittleren Bereich der Aufweitung haben. Die Richtung des Verbindens kann eine axiale Richtung des Rohrstücks, mit welchem die Aufweitung verbunden ist, sein.

Die Aufweitung kann ihren größten Querschnittsdurchmesser oder ihren größten Umfang in einem Querschnitt in einer Ebene senkrecht zu einer Erstreckungsrichtung des Innenlumens jenes Rohrstücks, mit welchem die Aufweitung verbunden ist, in einem in Verbindungsrichtung mittleren Bereich der Aufweitung haben.

Die Aufweitung kann in wenigstens drei unterschiedlichen Schnittebenen durch einen zentralen Punkt der Aufweitung oder in wenigstens drei unterschiedlichen Schnittebenen, welche eine gemeinsame Gerade umfassen, den gleichen Durchmesser und/oder Umfang aufweisen.

Der Durchmesser oder der Querschnittsdurchmesser der Aufweitung kann kreisförmig sein.

Die Aufweitung kann derart ausgestaltet sein, dass sie beim Verbinden des ersten Rohrstücks mit dem zweiten Rohrstück entlang eines Verschiebewegs im Rohrstück, in welches sie eingeführt wird, unter Beibehaltung des durch sie bewirkten Abdichtzustands bewegbar ist, und/oder derart ausgestaltet ist, dass sie beim Verbinden des ersten Rohrstücks mit dem zweiten Rohrstück oder nach Abschluss des Verbindens um einen Winkel bezogen zur Strömungsrichtung neigbar ist, unter Beibehaltung des durch sie bewirkten Abdichtzustands. Die verbundenen Rohrstücke können daher im Verbindungszustand etwas weiter ineinander geschoben oder auseinander herausgezogen werden, ohne dass der Verbindungszustand, welcher mittels der Aufweitung erzielt wurde, verloren gehen muss. Ebenso können die verbundenen Rohrstücke ein wenig zueinander geneigt oder in ihrer Verbindung miteinander abgeknickt werden ohne dass der Verbindungszustand verloren geht bzw. eine Undichtigkeit entsteht.

Die Aufweitung kann derart ausgestaltet sein, dass sie beim Verbinden des ersten Rohrstücks mit dem zweiten Rohrstück entlang eines Verschiebewegs im Rohrstücks, in welches sie eingeführt wird, unter Beibehaltung des durch sie bewirkten Abdichtzustands bewegbar ist. Der Verschiebeweg kann 1, 2, 3, 4, 5, 6, 7, 8, 9, 10 oder mehr Millimeter (mm) betragen.

Die Aufweitung kann derart ausgestaltet sein, dass sie beim Verbinden des ersten Rohrstücks mit dem zweiten Rohrstück oder nach Abschluss des Verbindens um einen Winkel bezogen zur Strömungsrichtung neigbar ist, unter Beibehaltung des durch sie bewirkten Abdichtzustands. Der Winkel kann in einem Bereich von 1 bis 3° liegen, er kann in einem Bereich von 1 bis 5°, 1 bis 8°, 1 bis 10° liegen. Er kann Zahlenwerte von 1, 2, 3, 4, 5, 6, 7, 8, 9, oder 10° oder mehr annehmen.

Die erfindungsgemäße Verbindungseinrichtung kann eine weitere, zweite Abdichteinrichtung aufweisen. Diese kann in einem Gebrauchszustand der Verbindungseinrichtung der Aufweitung benachbart angeordnet sein.

Die Verbindungseinrichtung kann ein Druckbegrenzungsventil aufweisen. Sie kann einen Abwasserzweig aufweisen.

Die Verbindungseinrichtung kann eine Schwenklagerung zum lateralen und/oder horizontalen Ausrichten des Innenrohrs und/oder des Außenrohrs und/oder des Konnektionsrohrs zueinander aufweisen.

Die Verbindungseinrichtung kann ergänzend zu anderen Zentrier- oder Ausrichteinrichtungen eine Vorzentriereinrichtung zur - vertikalen und/oder lateralen - Ausrichtung des Innenrohrs und/oder des Außenrohrs und/oder des Konnektionsrohrs vor Herstellen der Fluidverbindung aufweisen.

Die Vorzentriereinrichtung kann wenigstens zwei federnd auslenkbare Zungen aufweisen.

Die Verbindungseinrichtung kann eine Berührschutzscheibe zum Verhindern einer Kontamination eines Inneren und/oder eines Berührens des Konnektionsrohrs durch einen Benutzer aufweisen.

Die Berührschutzscheibe kann durch eine Rastanordnung in der externen, medizintechnischen Funktionseinrichtung gehalten werden. Sie kann eine Mehrzahl biegbarer Segmente aufweisen. Eine Anzahl von biegbaren Segmenten kann dabei unabhängig von anderen Segmenten biegbar sein.

Die Verbindungseinrichtung kann eine Einrichtung aufweisen, welche beim Konnektieren derart mechanisch verändert und/oder bewegt wird, dass die erfolgte Konnektion nach Dekonnektion an der Einrichtung erkennbar bleibt. Diese Einrichtung kann die Berührschutzscheibe sein.

Die Verbindungseinrichtung kann eine Faltenbalganordnung aufweisen. Diese kann Teil des Außenrohrs sein. Das Außenrohr kann wenigstens ein Federelement zum Straffen der Faltenbalganordnung aufweisen.

Die Verbindungseinrichtung kann eine Einrichtung zum Bewegen der Verschlusseinrichtung, beispielsweise einer abnehmbaren Spülkappe, aufweisen, zum Freigeben einer Öffnung des Innenrohrs zum Herstellen der Fluidverbindung. Die Spülkappe kann mittels der Einrichtung automatisch zum Freigeben bewegbar sein.

Das Innenrohr und das Außenrohr können axial zueinander verschiebbar angeordnet sein. Die Verbindungseinrichtung kann eine - insbesondere automatische - Einrichtung zum axialen Verschieben des Innenrohrs und/oder des Außenrohrs zueinander aufweisen.

Die Verbindungseinrichtung kann eine Einrichtung zum axialen Verschieben sowohl des Innenrohrs als auch des Außenrohrs aufweisen. Bei der hiermit erzielbaren Verschiebung kann die relative Lage von Innenrohr zu Außenrohr unverändert bleiben oder sich verändern.

Den Strömungsbereich der zu erzielenden Fluidverbindung mit ausgestaltende Werkstoffe können hydrophil sein. Alternativ oder ergänzend hierzu können Oberflächen im Strömungsbereich hydophil beschichtet sein.

Die erfindungsgemäße Verbindungseinrichtung kann beispielsweise als automatischer Substituat-Konnektor eingesetzt werden.

Die Kopplung kann über eine Kulissenführung erfolgen oder durch die Ausnutzung der Tatsache, dass die Spülkappe immer dann geschwenkt wird, wenn die Verbindungseinrichtung in Schwenkposition steht.

Auch eine Realisierung mit anderen Antriebsarten (mechanisch z.B. Zahnstange / Ritzel oder pneumatisch) ist von der Erfindung umfasst.

Die erfindungsgemäße Verbindungseinrichtung und/oder die erfindungsgemäße medizintechnische Vorrichtung können Sensoren zum Erfassen von Positionen oder Lagen von einzelnen Elementen der Verbindungseinrichtung (beispielsweise des Innenrohrs, des Außenrohrs, der Spülkappe, des Konnektionsrohrs, usw.) aufweisen.

Auch die verwendete Sensorik kann auf verschiedenen Prinzipien beruhen (axiale Positionserfassung z.B. durch Lichtschranken oder Erfassung der Spindeldrehung, Detektion der Schwenkbewegung ebenfalls mit Lichtschranken, durch Schalter, über Drehpotentiometer und dergleichen).

Die Flüssigkeit, die bei konnektiertem Disposable durch die erfindungsgemäße Verbindungseinrichtung strömt, kann zunächst zum Füllen und Spülen der externen Funktionseinrichtung, während der Behandlung dann als Substituat eingesetzt werden. Eine vollautomatische Konnektion, z.B. eines Dialysators (oder eines Rinseports) ist ebenfalls von der Erfindung umfasst.

Die erfindungsgemäße Aufgabe wird gleichermaßen durch eine erfindungsgemäße externe, medizintechnische Funktionseinrichtung gemäß Anspruch 14 gelöst. Alle mit der erfindungsgemäßen Verbindungseinrichtung erzielbaren Vorteile lassen sich ungeschmälert auch mit der erfindungsgemäßen externen Funktionseinrichtung erreichen.

Eine "externe Funktionseinrichtung" kann eine Einmalkomponente oder ein Einmalartikel sein. Sie kann aus einem Kunststoffmaterial gefertigt sein.

Die erfindungsgemäße externe Funktionseinrichtung kann zur Verwendung in einem Behandlungsverfahren vorgesehen sein. Behandlungsverfahren im Sinne der vorliegenden Erfindung schließen medizinische oder medizintechnische Behandlungsverfahren sowie Analyseverfahren der Labortechnik ein.

In einer bevorzugten Ausführungsform ist die erfindungsgemäße externe Funktionseinrichtung als Blutkassette ausgestaltet.

Eine Blutkassette im Sinne der vorliegenden Erfindung ist beispielsweise in den von der Anmelderin der vorliegenden Erfindung beim DPMA am 23. April 2009 bzw. 10. Juni 2009 eingereichten Anmeldungen 10 2009 018 664 A1 bzw. 10 2009 024 468 A1, jeweils mit dem Titel *"Externe Funktionseinrichtung, Blutbehandlungsvorrichtung zum Aufnehmen einer erfindungsgemäßen externen Funktionseinrichtung, sowie Verfahren"* beschrieben, auf deren diesbezügliche Offenbarungen hiermit vollinhaltlich Bezug genommen wird.

Die erfindungsgemäße Aufgabe wird gleichermaßen durch ein erfindungsgemäßes Verfahren gemäß Anspruch 12 gelöst. Alle Vorteile der erfindungsgemäßen Verbindungseinrichtung lassen sich ungeschmälert auch mit dem erfindungsgemäßen Verfahren erreichen.

Beim erfindungsgemäßen Verfahren zum Konnektieren von wenigstens zwei fluidführenden medizintechnischen Systemen, wird wenigstens eine erfindungsgemäße Verbindungseinrichtung verwendet.

Beim Verfahren kann ein Aufweiten des Innenlumens des Konnektionsrohrs mittels der Aufweitung des Innenrohrs erfolgen, wobei eine fluiddichte Verbindung erzielt wird.

Das Verfahren kann ferner ein Durchführen einer automatischen Routine zum Feststellen etwaiger Undichtigkeiten der mittels der Verbindungseinrichtung erzielten Fluidverbindung und/oder der Funktion eines Druckbegrenzungsventils der Verbindungseinrichtung umfassen.

Beim erfindungsgemäßen Verfahren kann die Spülkappe automatisch verschwenkt werden, um das Innenrohr zum Herstellen der Fluidverbindung freizugeben.

Die erfindungsgemäße Aufgabe wird auch durch eine erfindungsgemäße Behandlungsvorrichtung gemäß Anspruch 15 gelöst. Alle Vorteile der erfindungsgemäßen Verbindungseinrichtung lassen sich wiederum ungeschmälert mit der erfindungsgemäßen Behandlungsvorrichtung erreichen.

In einer bevorzugten Ausführungsform der vorliegenden Erfindung ist die Behandlungsvorrichtung eine Blutbehandlungsvorrichtung, wie eine Dialysiervorrichtung zum Durchführen einer Dialysebehandlung, wie einer Hämodialyse, Hämofiltration, Hämodiafiltration und dergleichen.

Die Behandlungsvorrichtung kann gleichermaßen zum Durchführen einer Peritonealdialyse geeignet sein.

Die erfindungsgemäße Verbindungseinrichtung kann dazu dienen, vorteilhaft automatisch eine Konnektion zwischen zwei fluidführenden Systemen, insbesondere für eine Flüssigkeit, herzustellen.

Dies kann den Vorteil bieten, dass der Benutzer während der Konnektion prinzipiell nicht in Kontakt mit fluid- oder flüssigkeitsberührenden Teilen kommt. Auf diese Weise kann vorteilhaft eine Kontamination der Verbindungsstelle und/oder des geförderten Fluids während des Konnektionsvorgangs verringert oder ausgeschlossen werden.

Daneben kann der Benutzer vorteilhaft entlastet werden, eine Schulung der Benutzer kann entfallen. Ferner entfällt die Gefahr einer verkehrten oder gar Schaden anrichtenden Konnektion.

Der Konnektionsvorgang kann in einen automatisierten Gesamtvorgang eingebunden werden, der ohne Benutzereingriff ablaufen kann (Bsp: Initialtest der Maschine mit anschließendem Füllen/Spülen).

Durch konzentrisches Anordnen des Innenrohrs der Verbindungseinrichtung kann die Konnektionsstelle vorteilhaft steril oder hygienisch gehalten werden.

Mit dem erfindungsgemäßen Verfahren kann es vorteilhaft möglich sein, die Behandlungsvorrichtung und die externe Funktionseinrichtung gleichzeitig keimfrei und/oder fremdstofffrei zu halten. Eine gegenseitige Verschmutzung kann vermieden werden.

Bei der erfindungsgemäßen Verbindungseinrichtung kann in vorteilhafter Weise die - für Sterilisationsverfahren unter Gaseinsatz gewöhnlich erforderliche - im Wesentlichen vollständige und druckverlustarme Zugänglichkeit aller zu sterilisierenden Oberflächen der externen Funktionseinrichtung sichergestellt werden.

Durch automatisches und/oder automatisiertes Verschließen der Verbindungseinrichtung kann zudem vorteilhaft ein Auslaufen von Restflüssigkeitsmengen aus den Konnektionsmündungen nach Ende einer Behandlungsprozedur vermieden werden.

Durch Einsatz der hierin beschriebenen Berührschutzscheibe kann die erfindungsgemäße Verbindungseinrichtung und/oder die erfindungsgemäße externe Funktionseinrichtung vorteilhaft mit einer Wiederverwendungssperre ausgerüstet werden. Diese stellt insbesondere einen Originalitätsschutz dar, welcher vorteilhaft in leicht erkennbarer Form anzeigt, dass die externe Funktionseinrichtung bereits einmal verwendet worden ist.

Mit der erfindungsgemäßen Verbindungseinrichtung kann es vorteilhaft möglich sein, möglichst vollständige Befüllungen und/oder Entleerungen durch strömungsgerechte Gestaltung und Anordnung aller Fluid führender Bereiche (auch kurz: Fluidbereiche), insbesondere des Innenrohrs, des Außenrohrs und des Konnektionsrohrs, zu gewährleisten. Auf diese Weise kann vorteilhaft auch sichergestellt werden, dass eine möglichst geringe Restmengen an Flüssigkeit im Fluidsystem der verwendeten Behandlungsvorrichtung verbleibt und gar keine oder nur sehr wenig Flüssigkeit in die Umgebung der Konnektionsmündung austritt.

Da das Innenlumen des Innenrohrs zum Zeitpunkt des Entfernens der Spülkappe teilentleert oder völlig ohne Flüssigkeitsinhalt sein kann, kann auf diese Weise vorteilhaft die Gefahr verringert oder sogar vermieden werden, dass umherfliegende Flüssigkeitströpfchen, welche bei der Entfernung der Spülkappe im letzten Moment noch Kontakt mit unsterilen Zonen des Außenrohrs und/oder der Spülkappe haben könnten, sich mit sterilen Restflüssigkeiten in der Verbindungseinrichtung mischen können und so bei erneuter Konnektion mit dem Verbindungspartner in das Innenlumen des Innenrohrs verschleppt werden könnten.

Ferner kann die erfindungsgemäße Verbindungseinrichtung ebenso vorteilhaft die Freiheit von Totraumstellen und/oder stationären Wirbeln gewährleisten. Dies kann vor allem bei der Sterilisation von Vorteil sein.

Bei einer horizontalen Konnektionsanordnung bleiben das Innen- und das Außenlumen nach der Spülung i.d.R. mit Spüllösung gefüllt. Dabei besteht die Gefahr, dass zumindest geringe Mengen an Flüssigkeit bei jedem manuellen Entfernen der Verschluss- oder Spülkappe in die Umgebung gelangen. Dies kann mit der erfindungsgemäßen Verbindungseinrichtung vorteilhaft vermieden werden.

Bei Verwendung der Verschluss- oder Spülkappe der erfindungsgemäßen Verbindungseinrichtung können erfindungsgemäß vorteilhaft Nebenräume vermieden werden. Dies erlaubt eine vereinfachte und besonders gründliche Desinfektion und Spülung.

Des Weiteren ist die Spülkappe erfindungsgemäß vorzugsweise innen strömungsgünstig ausgelegt, so dass bei Desinfektion und Spülung vorteilhaft alle Strömungsbereiche ausreichend gespült werden können.

Durch entsprechende - z.B. kegelförmige - Ausgestaltung des Innenrohrs, kann weiter vorteilhaft sichergestellt werden, dass kritische Dichtbereiche besonders intensiv desinfiziert und gespült werden. Durch die aufgrund der Ausgestaltung erzielbare Rotationsströmung können selbst enge Fugen erreicht und dabei auch die strömungstechnischen Vorraussetzungen für den Rückfluss der Fluide über das Außenlumen geschaffen werden.

Die erfindungsgemäße Verbindungseinrichtung kann in vorteilhafter Weise eine möglichst berührsichere und nach Möglichkeit sogar hustgeschützte Ausführung aller relevanten Bereiche bereitstellen.

Die erfindungsgemäße Verbindungseinrichtung kann ferner vorteilhaft besonders toleranzintolerant ausgestaltet sein. Dies gilt vorteilhaft sowohl für Toleranzen innerhalb der einzelnen Teilanordnungen von Behandlungsvorrichtung und externer Funktionseinrichtung als auch für die Toleranzenverträglichkeit der beiden Konnektionspartner bzw. Fluidsysteme untereinander. Sowohl bei manueller als auch bei maschineller Durchführung der eigentlichen Konnektion ist es somit vorteilhaft möglich, eine möglichst große - insbesondere laterale - Toleranz der Konnektionspartner vor und während der Konnektion zu verkraften und auch in Richtung der Konnektion eine möglichst große Toleranz für die Schiebebewegungen zuzulassen. Als Kombination beider Toleranzen kann in vorteilhafter Weise eine ausreichende Winkeltoleranz zwischen den Konnektionsachsen der beiden Konnektionspartner vorliegen.

Während der Konnektion stellt die erfindungsgemäße Verbindungseinrichtung vorteilhaft eine ausreichende Dichtheit bis zum Ende der Behandlung bereit. Dem Fall einer dennoch auftretenden Undichtigkeit kann die erfindungsgemäße Verbindungseinrichtung vorteilhaft durch die zweite redundante Dichtung, den Redundanzdichtwulst, entgegenwirken. Zudem kann erfindungsgemäß vorteilhaft Leckageflüssigkeit detektiert und kontrolliert abgeleitet werden.

Um einer mechanischen Querkraftbelastung zwischen den Konnektionspartnern während der Konnektion entgegenzuwirken, sieht die erfindungsgemäße Verbindungseinrichtung vorteilhaft eine querkraftarme Auslegung der Anordnung insbesondere bei gleichzeitigem Einfluss der Schwerkraft und bei Ausnutzung der maximalen Lateraltoleranz vor.

Mit der erfindungsgemäßen Verbindungseinrichtung wird die Behandlungsvorrichtung vorteilhaft vor dem ungewollten Zugriff auf die Konnektionsbereiche geschützt, so dass Wartung und/oder Inspektion dieser Bereiche vorteilhaft reduziert werden können. Auf diese Weise kann es vorteilhaft möglich sein, die zur Wartung der Behandlungsvorrichtung erforderlichen Kosten zu verringern.

Zudem können alle für die Konnektionsanordnung relevanten Komponenten der Behandlungsvorrichtung vorteilhaft leicht inspiziert und/oder ausgewechselt werden.

Zum Erzielen einer erfindungsgemäßen Fluidkommunikation kann zudem vorteilhaft auf die Verwendung teurer Werkstoffe wie etwa Silikongummi verzichtet werden. Auf diese Weise können die Herstellungskosten vorteilhaft reduziert werden.

Ferner können mit der erfindungsgemäßen Verbindungseinrichtung im Bereich der Behandlungsvorrichtung Elastomerdichtungen vermieden werden. Auf diese Weise können vorteilhaft ansonsten schlecht durchspülbare Grenz- und/oder Totraumzonen zwischen den Elastomerdichtungen und ihren Einbauräumen vermieden werden. Durch Ersetzen der vormais abrasiv bei den Konnektionen beanspruchten Elastomerdichtungen durch verschleißarme nichtelastomere Dichtanordnungen können ferner Wartungszyklen in vorteilhafter Weise deutlich verlängert werden.

Ferner ist, wenn die erfindungsgemäße Verbindungseinrichtung auf Seiten einer externen Funktionseinrichtung vorgesehen ist, das Außenrohr nach Abnahme der Spül- bzw. Schutzkappe vorteilhaft ebenso berührgeschützt, wie es das Außenrohr der Verbindungseinrichtung auf Seiten der Behandlungsvorrichtung ist. Auf diese Weise kann ebenso vorteilhaft das Verschleppen oder Einschleppen von Keimen bei der Konnektion mit der Behandlungsvorrichtung in das Innenrohr vermieden werden.

Eine erste Ausführungsvariante stellt eine automatisch konnektierende Anordnung zwischen einer externen Funktionseinrichtung und einer Behandlungsvorrichtung dar.

Die erfindungsgemäße Verbindungseinrichtung ist hierbei üblicherweise als Teil der Behandlungsvorrichtung ausgestaltet und vorgesehen.

Eine derartige Anordnung kann besonders vorteilhaft verwendbar sein bei Verbindungen zwischen externen Funktionseinrichtungen und Behandlungsvorrichtungen, bei denen mehrere räumlich definierte Verbindungen zwischen externer Funktionseinrichtung und Behandlungsvorrichtung gleichzeitig zustande kommen sollen oder müssen.

Eine derartige Anordnung kann beispielsweise zur automatischen Substituatkonnektion einer externen Funktionseinrichtung, wie beispielsweise einer Blutkassette, im Rahmen einer Dialysebehandlung erfolgen. Ferner kann diese Anordnung auch für eine automatische Hämofilter-Dialysatkonnektion aktueller und/oder zukünftiger Kassetten-Dialysesysteme eingesetzt werden.

Eine zweite Ausführungsvariante stellt eine automatisch konnektierende Anordnung zwischen einer ersten externen Funktionseinrichtung und einer zweiten externen Funktionseinrichtung dar.

Im Gegensatz zu der ersten Ausführungsvariante, in der die erfindungsgemäße Verbindungseinrichtung Teil einer Behandlungsvorrichtung ist, ist die erfindungsgemäße Verbindungseinrichtung hier vorwiegend als Einmalteilanordnung an einer externen Funktionseinrichtung ausgeführt.

Derartige Anordnungen können beispielsweise als automatisches Konnektionssystem für alle Beutel-Behandlungsflüssigkeiten zur Behandlungskassette bei Akut-Hämodialysemaschinen und bei Peritonealdialysemaschinen zukünftiger Generationen eingesetzt werden.

Eine dritte Ausführungsvariante umfasst manuell konnektierende Anordnungen zwischen einer externen Funktionseinrichtung und einer Behandlungsvorrichtung.

Bei einer derartigen Anordnung kann der Antrieb zum automatischen oder automatisierten Konnektieren der beiden fluidführenden Systeme entfallen oder durch ein ähnliches System mit manueller Betätigung, beispielsweise in Form eines Hebels zum Antrieb des Mechanismus, ersetzt werden.

Zudem kann, wenn nur je ein räumlich frei bewegliches Konnektionsrohr einer externen Funktionseinrichtung, d.h. ein Disposablekonnektor (etwa Konnektor an einem Schlauch), an die Behandlungsvorrichtung angeschlossen wird, ein Teil des Toleranzausgleichssystems entfallen (Schwenkanordnung und Vorzentrierung der Verbindungseinrichtung).

Derartige Anordnungen können beispielsweise zur Rinse-Konnektion und/oder SubstituatKonnektion bei Schlauchsystem-Dialysebehandlungsvorrichtungen, Rinse-Konnektion bei aktuellen Blutkassetten und dergleichen eingesetzt werden.

Eine vierte Ausführungsvariante schließt manuell konnektierende Anordnungen zwischen wenigstens zwei externen Funktionseinrichtungen ein.

Um die Konnektionskräfte manuell beherrschbar zu machen, können zusätzliche kraftverstärkende Konsiruktionselemente wie Exzenterhebel und/oder Überwurfmuttern und dergleichen hinzutreten. Bei derartigen Anordnungen kann die Verwendung nachgiebiger und/oder gleitgünstiger Einmalteil-Werkstoffe sinnvoll sein.

Im Folgenden wird die vorliegende Erfindung jeweils beispielhaft unter Bezugnahme auf die beigefügte Zeichnung beschrieben. In der Zeichnung bezeichnen identische Bezugszeichen gleiche oder identische Elemente. Es gilt:
- Fig. 1: zeigt eine erfindungsgemäße Verbindungseinrichtung im Längsschnitt;
- Fig. 2: zeigt eine Grundstellung einer Konnektorbaugruppe einer externen Funktionseinrichtung im Längsschnitt;
- Fig. 3: zeigt eine Grundstellung einer erfindungsgemäßen Verbindungseinrichtung im Längsschnitt;
- Fig. 4: zeigt einen vergrößerten Abschnitt der Verbindungseinrichtung aus Fig. 3;
- Fig. 5: zeigt eine erfindungsgemäße Verbindungseinrichtung in einem Teillängsschnitt;
- Fig. 6: zeigt eine perspektivische Ansicht der erfindungsgemäßen Verbindungseinrichtung als Teil einer Behandlungsvorrichtung;
- Fig. 7: zeigt eine einen Zustand während des Herstellens einer Fluidkommunikation zwischen einer externen Funktionseinrichtung und einer Behandlungsvorrichtung mittels der erfindungsgemäßen Verbindungseinrichtung;
- Fig. 8: zeigt die erfindungsgemäße Verbindungseinrichtung in einem ersten Zustand während des Herstellens einer Fluidkommunikation im Längsschnitt;
- Fig. 9: zeigt die erfindungsgemäße Verbindungseinrichtung in einem zweiten Zustand während des Herstellens der Fluidkommunikation im Längsschnitt;
- Fig. 10: zeigt einen vergrößerten Abschnitt der erfindungsgemäßen Verbindungseinrichtung in einem dritten Zustand während des Herstellens der Fluidkommunikation;
- Fig. 11: zeigt die erfindungsgemäße Verbindungseinrichtung in einem vierten Zustand während des Herstellens der Fluidkommunikation im Längsschnitt;
- Fig. 12: zeigt die erfindungsgemäße Verbindungseinrichtung in einem fünften Zustand während des Herstellens der Fluidkommunikation im Längsschnitt;
- Fig. 13: zeigt eine weitere perspektivische Ansicht der erfindungsgemäßen Verbindungseinrichtung als Teil einer Behandlungsvorrichtung;
- Fig. 14: zeigt die erfindungsgemäße Verbindungseinrichtung in einem ersten Zustand einer Fluidkommunikation im Längsschnitt;
- Fig. 15: zeigt die erfindungsgemäße Verbindungseinrichtung in einem zweiten Zustand der Fluidkommunikation im Längsschnitt;
- Fig. 16: zeigt eine weitere perspektivische Ansicht der erfindungsgemäßen Verbindungseinrichtung als Teil einer Behandlungsvorrichtung;
- Fig. 17: zeigt eine weitere perspektivische Ansicht der erfindungsgemäßen Verbindungseinrichtung als Teil einer Behandlungsvorrichtung;
- Fig. 18: zeigt eine perspektivische Ansicht auf eine erfindungsgemäße Verbindungseinrichtung in einer Schwenkposition.
- Fig. 19A: zeigt die Berührschutzscheibe vor Gebrauch in perspektivischer Ansicht;
- Fig. 19B: zeigt die Berührschutzscheibe nach Gebrauch in perspektivischer Ansicht;
- Fig. 20A: zeigt eine Spülkappe mit einer Spiralstruktur in perspektivischer Ansicht;
- Fig. 20B: zeigt eine Spülkappe in axialdichtender Bauform im Längsschnitt;
- Fig. 21A: zeigt eine Verschlusshülse in einer Bauform A im Längsschnitt;
- Fig. 21B: zeigt eine Verschlusshülse in einer Bauform B mit verbessertem Tropfschutz in perspektivischer Ansicht;
- Fig.22A: zeigt einen Längsschnitt der Verschlusshülse der Bauform B in einer Grundstellung;
- Fig.22B: zeigt einen Längsschnitt der Verschlusshülse der Bauform B in einer Verschlussstellung;
- Fig. 23A: zeigt einen Längsschnitt eines Lateralausgleichs in einer Bauform A in einer Grundstellung;
- Fig. 23B: zeigt einen Längsschnitt des Lateralausgleichs der Bauform A in einer Auslenkstellung;
- Fig. 24A: zeigt einen Längsschnitt eines Lateralausgleichs in einer Bauform B in einer Grundstellung;
- Fig. 24 B: zeigt einen Längsschnitt des Lateralausgleichs der Bauform B in einer Vorzentrierstellung;
- Fig. 24 C: zeigt einen Längsschnitt des Lateralausgleichs der Bauform B in einer Konnektionssteilung;
- Fig. 25A: zeigt einen Längsschnitt einer Ausführungsform der erfindungsgemäßen Verbindungseinrichtung während einer Desinfektion, eines Spülvorgangs und/oder in Bereitschaft;
- Fig. 25B: zeigt einen Längsschnitt einer Ausführungsform der erfindungsgemäßen Verbindungseinrichtung während eines Leerblasens und/oder zu Beginn eines Konnektionsvorgangs;
- Fig. 25C: zeigt einen Längsschnitt einer Ausführungsform der erfindungsgemäßen Verbindungseinrichtung während der Konnektion und/oder eines Verschließvorgangs;
- Fig. 26: zeigt einen Längsschnitt eines Dialysefilters mit einer Konnektorbaugruppe im Dialysatanschluss;
- Fig. 27: zeigt einen Flussplan einer erfindungsgemäßen Behandlungsvorrichtung, die eine erfindungsgemäße Verbindungseinrichtung und eine externe Funktionseinrichtung aufweist; und
- Fig. 28: zeigt einen Rinse-Port im Längsschnitt.

Im Folgenden wird die Erfindung mit Bezug auf eine Behandlungseinrichtung erklärt. Dabei ist die Erfindung oder ihre Ausgestaltung nicht auf eine Behandlungseinrichtung beschränkt. Alles im Zusammenhang mit einer Behandlungsvorrichtung Gesagte gilt daher allgemein für medizintechnische Vorrichtungen, wie z.B. solche aus der Therapie, Diagnose, Analyse, usw.

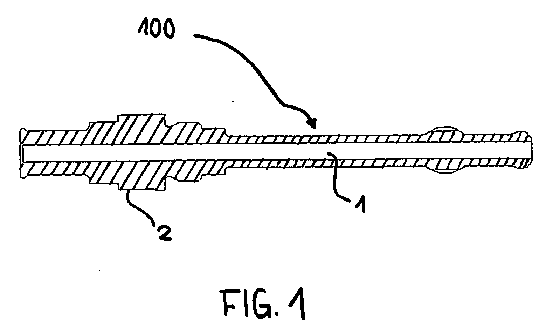

**Fig. 1** zeigt eine erfindungsgemäße Verbindungseinrichtung 100 im Längsschnitt.

Die Verbindungseinrichtung 100 weist ein Innenrohr 1 auf, welches - nur vorzugsweise konzentrisch - in einem Außenrohr 3 angeordnet ist.

Das Innenrohr 1 kann axial in dem Außenrohr 3 bewegbar sein.

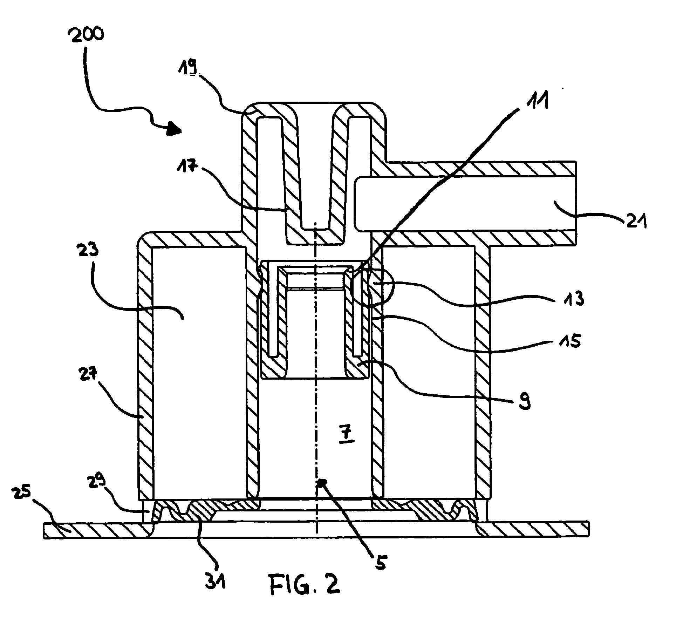

Die Fig. 2 bis 4 zeigen jeweils eine Grundstellung von Anordnungen bzw. Baugruppen, die zum Herstellen einer Fluidverbindung gemäß dem erfindungsgemäßen Verfahren oder mittels der erfindungsgemäßen Verbindungseinrichtung vorbereitet sind.

**Fig. 2** zeigt einen Längsschnitt durch eine Konnektorbaugruppe 200 zur Verbindung mit einer in Fig. 2 nicht gezeigten erfindungsgemäßen Verbindungseinrichtung.

Die Konnektorbaugruppe 200 ist hier Teil einer externen Funktionseinrichtung, während die Verbindungseinrichtung Teil einer Behandlungsvorrichtung ist. Allerdings könnten die Konnektorbaugruppe 200 auch an der Behandlungsvorrichtung, die Verbindungseinrichtung hingegen an der externen Funktionseinrichtung vorliegen.

Die Konnektorbaugruppe 200 weist ein Konnektionsrohr 5 mit einem Innenlumen 7 auf.

Im Inneren des Konnektionsrohrs 5 ist eine Verschlusshülse 9 angeordnet. Die Verschlusshülse 9 weist an ihrem oberen Ende (bezogen auf ihre Ausrichtung in Fig. 2) einen Verschlusskragen 11 auf.

Die Verschlusshülse 9 wird durch eine Rastanordnung 13 in einer Innenwandung 15 des Konnektionsrohrs 5 in ihrer Position gegen eine Verschiebung - z. B. nach unten - gehalten.

Die Rastanordnung 13 weist eine Verdickung in der Innenwandung 15 des Konnektionsrohrs 5 und eine Aussparung oder Vertiefung in einer Seitenwand der Verschlusshülse 9 auf. Von der Erfindung umfasst sind aber auch umgekehrte Anordnungen, nämlich Vertiefung in der Innenwand und Verdickung in der Verschlusshülse. Derartige "umgekehrte" Ausgestaltungen sind im Rahmen der hier beschriebenen Erfindung stets dann mitoffenbart, wenn sie für den Fachmann erkennbar technisch realisierbar sind.

Alternativ könnte die Rastanordnung 13 auch als eine nicht dargestellte Klemmeinrichtung oder dergleichen ausgestaltet sein.

An seinem oberen Ende (bezogen auf die Ausrichtung in Fig. 2) weist das Konnektionsrohr 5 einen Verschlusszapfen 17 auf. Der Verschlusszapfen 17 ist, wie in Fig. 2 gezeigt, zinnenförmig ausgebildet. An der Oberseite des Verschlusszapfens 17 ist ein Abstützdom 19 vorgesehen.

An der rechten Seite des Verschlusszapfens 17 (bezogen auf die Anordnung in Fig. 2) ist ein Kanal 21 vorgesehen. Der Kanal 21 kann zu einem Inneren der externen Funktionseinrichtung abgehen.

Die Konnektorbaugruppe 200 weist einen Aufnahmeraum 23 zum Aufnehmen von Fluiden auf.

An ihrer Unterseite (bezogen auf die Anordnung in Fig. 2) weist die Konnektorbaugruppe 200 einen Abstützring 25 auf.

Zwischen einer Außenwandung 27 der Konnektorbaugruppe 200 und dem Abstützring 25 ist eine Rastanordnung 29 für eine Berührschutzscheibe 31 vorgesehen.

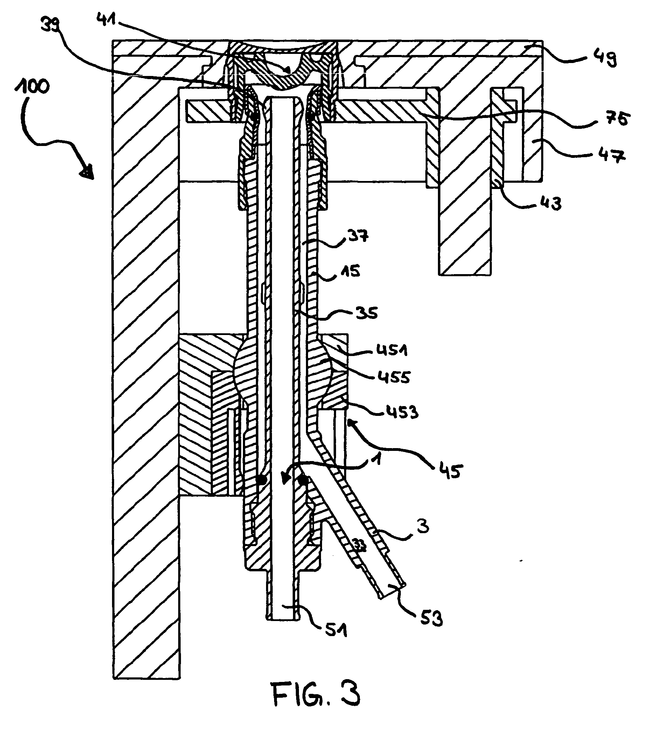

**Fig. 3** zeigt schematisch eine Verbindungseinrichtung 100 gemäß der vorliegenden Erfindung im Längsschnitt.

Die Verbindungseinrichtung 100 ist hier in ihrer Gesamtanordnung als Teil einer Behandlungsvorrichtung gezeigt.

Die Verbindungseinrichtung 100 weist ein Innenrohr 1 sowie ein Außenrohr 3 auf. Das Innenrohr 1 ist in das Außenrohr 3 eingeführt.

Das Innenrohr 1 weist ein Innenlumen 33 auf. Zwischen der Außenwandung 35 des Innenrohrs 1 und der Innenwandung 15 des Außenrohrs 3 ist ein Spalt vorhanden, welcher hier als ein Außenlumen 37 des Innenrohrs 1 bezeichnet wird.

Sowohl das Innenlumen 33 des Innenrohrs 1 als auch das Außenlumen 37 des Innenrohrs 1 sind zum Aufnehmen von Fluiden geeignet.

An seinem oberen Ende (bezogen auf die Ausrichtung in Fig. 3) weist eine Wand des Innenrohrs 1 eine Aufweitung 39 auf. Die Aufweitung 39 ist kugelig ausgeführt. Alternativ könnten sowohl das gesamte Innenrohr 1 also auch sein Innenlumen 33 eine derartige Verbreiterung aufweisen.

Die Verbindungseinrichtung 100 ist mit einer Spülkappe 41 bedeckt oder verschlossen.

Um die Spülkappe 41, beispielsweise zur Herstellung einer Fluidkommunikation mit einem weiteren fluidführenden System, automatisiert von der Verbindungseinrichtung 100 entfernen zu können oder umgekehrt, kann ein Antrieb vorgesehen sein. In Fig. 3 sind Teile eines solchen Antriebs in Form eines Spülkappen-Schwenkantriebs 43 gezeigt.

Der Spülkappen-Schwenkantrieb 43 weist eine Supporteinrichtung 45 auf.

Die Supporteinrichtung 45 weist ein Oberteil 451 und ein Unterteil 453 auf.

Das Oberteil 451 und das Unterteil 453 sind fest miteinander verbunden. In der Grundstellung in Fig. 3 sind das Oberteil 451 und das Unterteil 453 im Wesentlichen parallel oder spiegelsymmetrisch zueinander ausgerichtet. Das Oberteil 451 und das Unterteil 453 bilden zusammen einen Support (="Schlitten"), der kein Bestandteil des Schwenkantriebs ist. Im Support ist das Außenrohr mittels einer kugeligen bzw. umlaufend konvexen Auswölbung in einer entsprechenden "Pfanne" des Supports zu Toleranzausgleich in engen Grenzen verschwenkbar gegenüber dem Support gelagert.

Die Supporteinrichtung 45 weist eine Schwenklagerung 455 auf. Die Supporteinrichtung 45 wird mittels eines nicht gezeigten Linearantriebs bewegt.

Ferner ist in Fig. 3 ein Chassis 47 der Behandlungsvorrichtung gezeigt. Das Chassis 47 weist an seiner Oberseite (bezogen auf die Ausrichtung in Fig. 3) sowie an einer der Spülkappe 41 zugewandten Innenseite des Chassis 47 eine Gummiplatte 49 auf.

Das Innenrohr 1 weist einen Fluidanschluss 51 zum Einbringen von Fluiden in das Innenlumen 33 des Innenrohrs 1 auf.

Das Außenrohr 3 weist einen Fluidanschluss 53 zum Einbringen von Fluiden in das Außenlumen 37 des Innenrohrs 1 oder zum Abführen aus diesem auf.

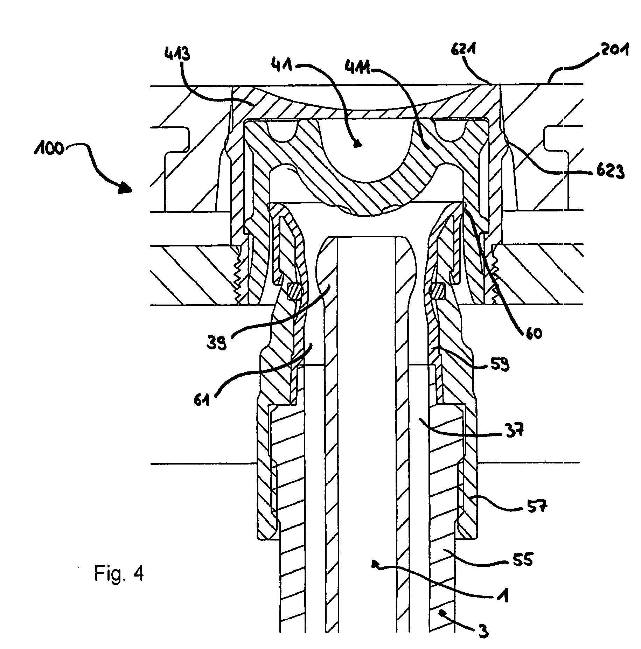

**Fig. 4** zeigt einen Abschnitt der erfindungsgemäßen Verbindungseinrichtung 100 aus Fig. 3 in einer Vergrößerung.

Der in Fig. 4 gezeigte Abschnitt oder Teilausschnitt der Verbindungseinrichtung 100 dient zum Herstellen einer Konnektion der Behandlungsvorrichtung und einer externen Funktionseinrichtung in Fluidverbindung.

Das Außenrohr 3 weist einen Sockelkörper 55 auf. Das Außenrohr 3 weist einen Kopfaufsatz oder -körper 57 auf. Das Außenrohr 3 weist zudem einen Dichtaufsatz oder - körper 59 auf.

Zwischen der Aufweitung 39 des Innenrohrs 1 und dem sich, wie in Fig.4 gezeigt, kelchförmig nach oben hin öffnenden Kopfkörper 57 des Außenrohrs 3 befindet sich ein Spalt 61.

Der Spalt 61 kann ein das Innenrohr 1 umlaufender Spalt sein. Er kann in jedem Abschnitt seines Umlaufs gleiche Abmessungen aufweisen. Er kann jedoch auch abschnittsweise verschiedene Abmessungen aufweisen, wenn das Innenrohr 1 nicht mittig in dem Außenrohr 3 angeordnet wäre.

Der Spalt 61 wird seitlich bzw. in radialer Richtung durch den Dichtkörper 59 des Außenrohrs 3 begrenzt. Unterhalb des Spalts 61 schließt sich das für Fluide zugängliche Außenlumen 37 des Innenrohrs 1 an.

Die Spülkappe 41 weist einen Fluideinsatz 411 sowie einen Montagekörper 413 auf.

Die Spülkappe 41 ist, wie in Fig. 4 gezeigt, auf bzw. über den Dichtkörper 59 des Außenrohrs 3 aufgesetzt bzw. über das Innenrohr 1 und das Außenrohr 3 gestülpt.

Der Dichtkörper 59 des Außenrohrs 3 wird an der Dichtstelle 60 zur Spülkappe 41 zusammengedrückt. Die Spülkappe 41 bzw. der Fluideinsatz 411 derselben wird an der Dichtstelle 60 aufgeweitet.

Die Spülkappe 41 ist an zwei umlaufenden Dichtstellen 621 und 623 gegenüber der Behandlungsvorrichtung bzw. einer Behandlungsvorrichtungsfront abgedichtet.

Die Spülkappe 41 sitzt bündig in bzw. an einer Oberfläche 201 der Behandlungsvorrichtung.

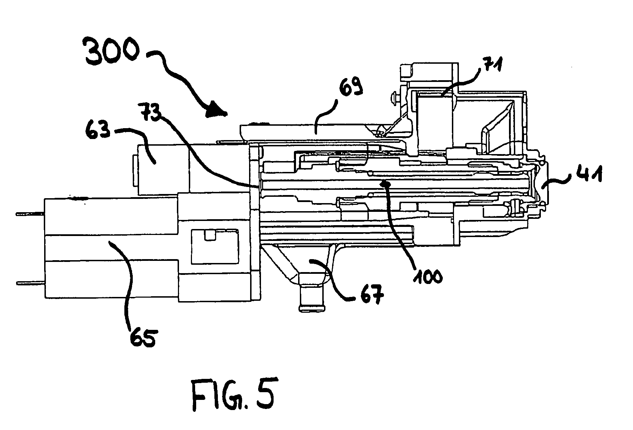

**Fig. 5** zeigt schematisch eine erfindungsgemäße Verbindungseinrichtung 100 als Teil einer Behandlungsvorrichtung 300 in einem Teillängsschnitt.

Die Verbindungseinrichtung 100 trägt in der in Fig. 5 gezeigten Grundstellung die Spülkappe 41.

Die Behandlungsvorrichtung 300 weist einen Motor, z.B. einen Gleichstrommotor 63, zum Schwenken der Spülkappe 41 auf.

Die Behandlungsvorrichtung 300 weist einen Motor, z.B. einen Gleichstrommotor 65, zum Verfahren der Verbindungseinrichtung 100 auf.

Die Benandlungsvorrichtung 300 weist einen Tropfwasserablauf 67 auf.

Die Behandlungsvorrichtung 300 weist einen Folienpotentiometer 69 zum Erkennen einer Position der Verbindungseinrichtung 100 auf.

Zur Erkennung einer Position der Spülkappe 41 kann die Behandlungsvorrichtung 300 mehrere und dabei ggf. auch verschiedenartige Sensoren aufweisen. Wie in Fig. 5 gezeigt ist, weist die Behandlungsvorrichtung 300 hierzu einen Hallsensor 71 auf.

Ein Zulauf 73 zum Einbringen von Fluiden in die Verbindungseinrichtung 100 führt in das Innere der Verbindungseinrichtung 100.

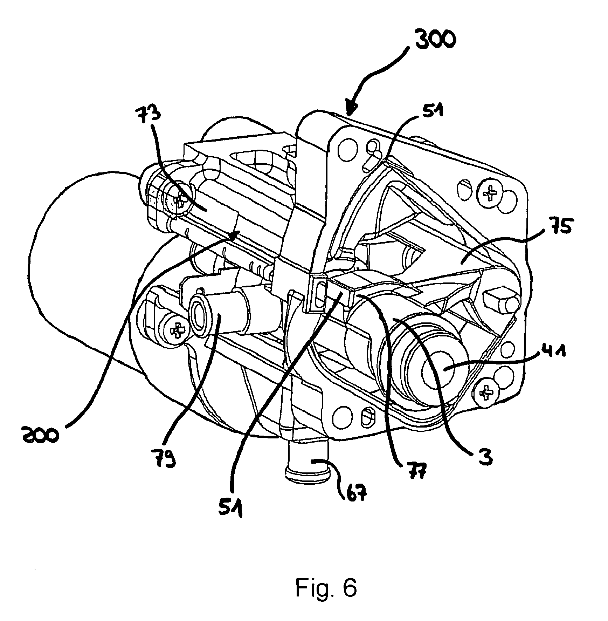

**Fig. 6** zeigt die erfindungsgemäße Verbindungseinrichtung 100 als Teil einer Behandlungsvorrichtung 300 von vorne und oben, von der Seite der anzukoppelnden bzw. anzubindenden externen Funktionseinrichtung aus. In der Darstellung der Fig. 6 ist ein vorderes Gehäuseteil der Behandlungsvorrichtung 300 entfernt.

Die Verbindungseinrichtung weist die Spülkappe 41 auf.

Der Zulauf 73 ist in der vorliegenden Darstellung der erfindungsgemäßen Verbindungseinrichtung 100 verdeckt. Durch Angabe des Bezugszeichens soll seine Lage angedeutet werden.

Die Behandlungsvorrichtung 300 weist einen Schwenkhebel 75 auf. Die Spülkappe 41 ist starr mit dem Schwenkhebel 75 verbunden.

Der Schwenkhebel 75 weist einen Magneten 77 auf.

In Fig. 6 ist die Behandlungsvorrichtung 300 mit zwei Hallsensoren 51 gezeigt.

Die Verbindungseinrichtung 100 ist mit einem Rohranschluss für einen Kanal (hier nicht gezeigt) zur externen Funktionseinrichtung versehen. Dieser Kanal kann beispielsweise als Rücklauf 79 für Fluide dienen.

**Fig**. **7** zeigt schematisch einen Zustand während des Herstellens einer Fluidkommunikation zwischen einer Behandlungsvorrichtung 300 und einer externen Funktionseinrichtung 400 mittels der erfindungsgemäßen Verbindungseinrichtung 100. Die Anordnung ist im Längsschnitt dargestellt.

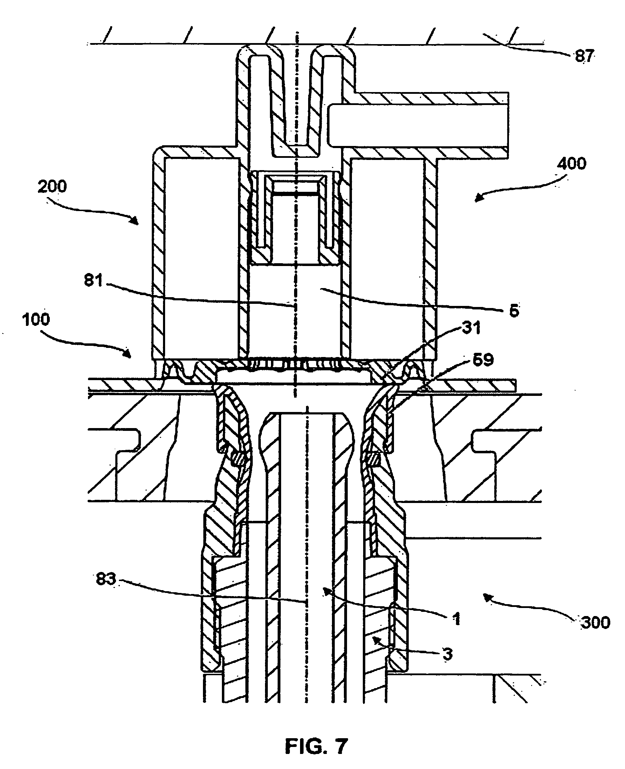

In Fig. 7 ist die externe Funktionseinrichtung 400 gerade an die Behandlungsvorrichtung 300 angedockt. Fig. 7 zeigt sozusagen den Erstkontakt zwischen der Behandlungsvorrichtung 300 und der externen Funktionseinrichtung 400 beim Herstellen der Fluidverbindung.

Der Dichtkörper 59 des Außenrohrs 3 stößt gegen die Berührschutzscheibe 31 der Konnektorbaugruppe 200 der externen Funktionseinrichtung 400.

Eine Mittelachse 81 des Konnektionsrohrs 5 und eine Mittelachse 83 des Innenrohrs 1 liegen erkennbar versetzt zueinander. Das Konnektionsrohr 5 befindet sich daher noch nicht in der gewünschten bzw. angestrebten Position bezogen auf das Innenrohr 1 zum Herstellen der Fluidkommunikation.

Oberhalb der Konnektorbaugruppe 200 bzw. über der externen Funktionseinrichtung 400 ist in Fig. 7 eine Abdeckungseinrichtung 85, beispielsweise eine Türfläche oder -platte, vorgesehen.

Eine solche Abdeckungseinrichtung kann zum Beispiel zum Verpressen der externen Funktionseinrichtung in der Behandlungsvorrichtung eingesetzt werden, wie es beispielsweise in der von der Anmelderin der vorliegenden Anmeldung am 8. März 2009 beim DPMA hinterlegten Patentanmeldung 10 2009 012 633.3 (Vertreterakte FM19A24) mit dem Titel "Vorrichtung zum Verbinden *einer externen Funktionseinrichtung mit einer Anordnung, Anordnung aufweisend eine solche Vorrichtung und Verfahren zum Verbinden"* offenbart ist, auf deren diesbezügliche Offenbarung hiermit vollinhaltlich Bezug genommen wird.

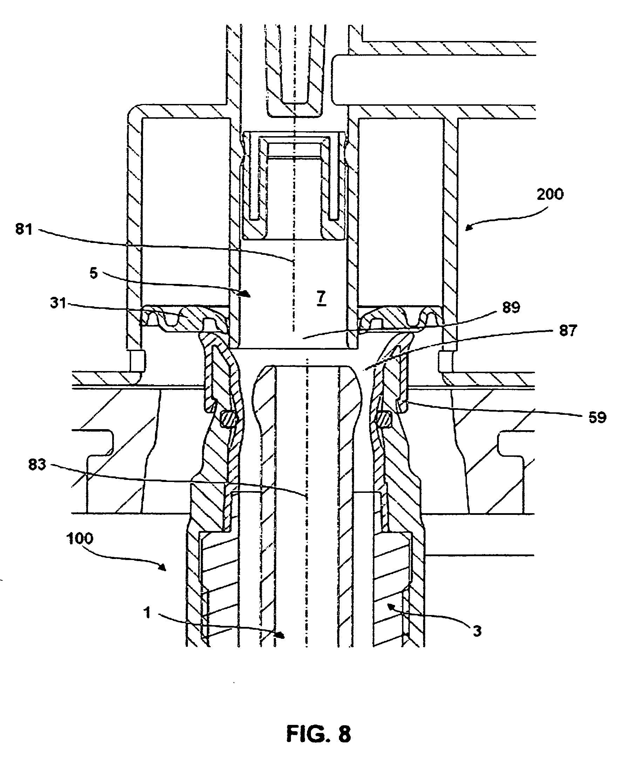

**Fig. 8** zeigt schematisch einen Längsschnitt durch die Verbindungseinrichtung 100 zu Beginn des Einfädelns des Konnektionsrohrs 5 in das Außenrohr 3.

Das Außenrohr 3 weist einen Einführtrichter 87 auf. Der Einführtrichter 87 ist Teil des Dichtkörpers 59 des Außenrohrs 3.

Wie in Fig. 8 gezeigt steht die Mittelachse 81 des Konnektionsrohrs 5 nicht mittig zum Außenrohr 3. Vielmehr ist zu erkennen, dass das Außenrohr 3 entlang der Unterseite der Konnektorbaugruppe 200 nach rechts verschoben ist oder umgekehrt.

Der Einführtrichter 87 des Außenrohrs 3 grenzt bzw. stößt an die Berührschutzscheibe 31 der Konnektorbaugruppe 200 an.

Die Mittelachse 83 des Innenrohrs 1 liegt versetzt zu der Mittelachse 81 des Konnektionsrohrs 5.

Das Konnektionsrohr 5 weist in seinem Innenlumen 7 eine laterale Toleranz 89 auf. Die laterale Toleranz 89 dient vorzugsweise dazu, ein gewisses Maß an Spiel, beispielsweise zum Ausgleich von Passungenauigkeiten, bereitzustellen.

Mittels der lateralen Toleranz 89 kann vorteilhaft ein leicht durchführbares und/oder beschädigungsfreies Einfädeln des Innenrohrs 1 in das Konnektionsrohr 5 erfolgen.

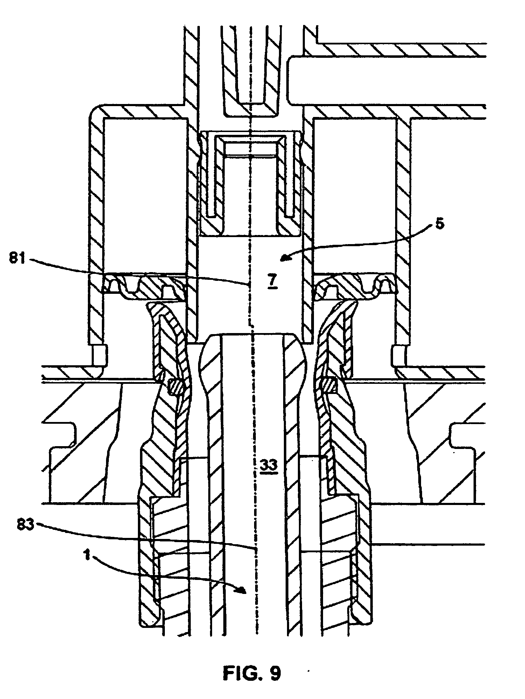

**Fig. 9** zeigt schematisch einen Längsschnitt durch die Verbindungseinrichtung 100 gegen Ende des Einfädelns des Außenrohrs 3.

Das Innenrohr 1 ist zum Teil in das Konnektionsrohr 5 eingeschoben oder eingeführt (oder umgekehrt). Das Innenlumen 33 des Innenrohrs 1 steht in Fluidkommunikation mit dem Innenlumen 7 des Konnektionsrohrs 5. Jedoch ist noch nicht die bevorzugte Anordnung zur Fluidverbindung erreicht.

Wie in Fig. 9 gezeigt ist, fluchtet das Innenrohr 1 noch nicht sauber mit dem Konnektionsrohr 5. Die Mittelache 83 des Innenrohrs 1 und die Mittelachse 81 des Konnektionsrohrs 5 sind zueinander versetzt.

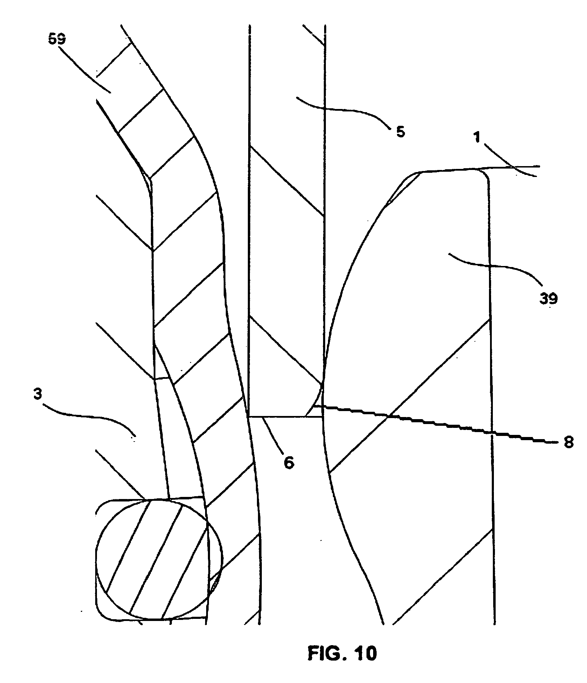

**Fig. 10** zeigt eine ausschnittsweise Vergrößerung einer Stellung zu Beginn des Einfädelns des Innenrohrs 1 in das Konnektionsrohr 5.

Während des Einfädelns des Innenrohrs 1 in das Konnektionsrohr 5 wird die Wandung des Konnektionsrohrs 5 zwischen die Aufweitung 39 des Innenrohrs 1 und den Dichtkörper 59 des Außenrohrs 3 geschoben.

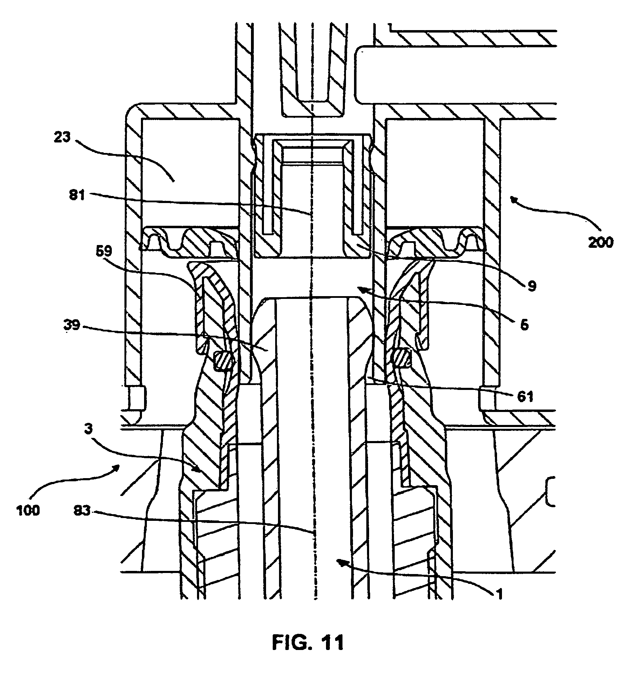

**Fig. 11** zeigt schematisch die Verbindungseinrichtung 100 in einer Stellung minimaler Konnektion des Innenrohrs 1 mit dem Konnektionsrohr 5.

Das Innenrohr 1 ist mit Aufweitung 39 und darüber hinaus in das Konnektionsrohr 5 eingeführt.

Die Wand des Konnektionsrohrs 5 füllt im Wesentlichen den Spalt 61 zwischen dem Dichtkörper 59 des Außenrohrs 3 und der Aufweitung 39 des Innenrohrs 1 aus.

Die Mittelachse 81 des Konnektionsrohrs 5 und die Mittelachse 83 des Innenrohrs 1 fallen nun zusammen.

Durch Ineinanderschieben des Innenrohrs 1, des Konnektionsrohrs 5 und des Außenrohrs 3 drückt die Aufweitung 39 das Konnektionsrohr 5 auseinander oder weitet dies zumindest lokal. Der Dichtkörper 59 des Außenrohrs 3 drückt von Außen gegen die Wand des Konnektionsrohrs 5.

Der Dichtkörper 59 des Außenrohrs 3 schiebt die Berührschutzscheibe 31 der Konnektorbaugruppe 200 in Fig. 11 nach oben, wodurch der Aufnahmeraum 23 der Konnektorbaugruppe 200 - beispielsweise im Vergleich zu dem in Fig. 2 gezeigten Aufnahmeraum - kleiner wird.

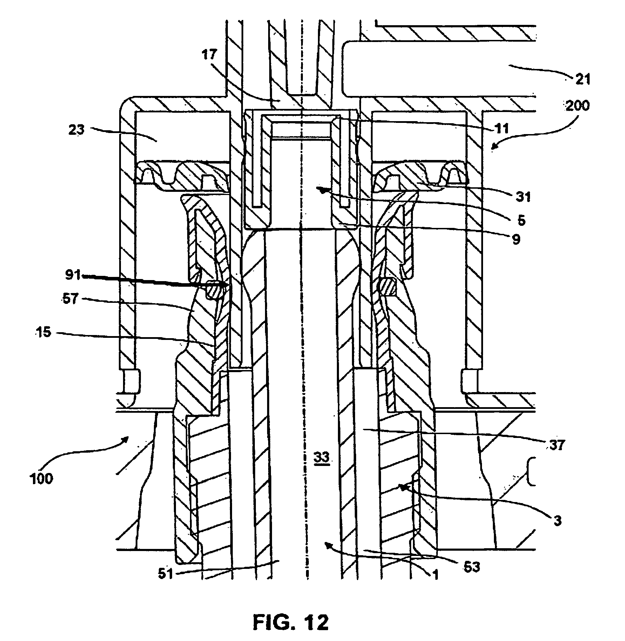

**Fig. 12** zeigt schematisch die Verbindungseinrichtung 100 in einer Stellung maximaler Konnektion des Innenrohrs 1 mit dem Konnektionsrohr 5.

Zwischen der minimalen Konnektionsstellung und der maximalen Konnektionsstellung ist jede axiale Stellung zulässig. Dies ergibt sich vorteilhaft aus dem in der erfindungsgemäßen Verbindungseinrichtung vorgesehenen axialen Toleranzausgleich.

In der in Fig. 12 gezeigten maximalen Konnektionsstellung oder Stellung maximaler Konnektion ist das Innenrohr 1 gegenüber der Stellung der Fig. 11 noch weiter in das Konnektionsrohr 5 hineingeschoben. Die Aufweitung 39 befindet sich vollständig im Inneren des Konnektionsrohrs 5.

Die Berührschutzscheibe 31 ist in Fig. 12 weiter nach oben verschoben, so dass der Aufnahmeraum 23 verglichen mit dem Aufnahmeraum aus Fig. 11 noch weiter verkleinert ist.

Die Stirnseite der Aufweitung 39 grenzt bereits vor Erreichen des Zustands der maximalen Konnektion direkt an die Verschlusshülse 9 der Konnektorbaugruppe 200 an und verschiebt dabei die Verschlusshülse 9 axial.

Der Verschlusskragen 11 grenzt an den Verschlusszapfen 17 der Konnektorbaugruppe 200 an.

Die fluidische Konnektion und/oder Dichtung kann dadurch zustande kommen, dass das Innenrohr 1 mit der Aufweitung 39 in das Konnektionsrohr 5 hineingeschoben wird. Da der Außendurchmesser der Aufweitung 39 größer ist als der Innendurchmesser des Konnektionsrohrs 5, wird das Konnektionsrohr 5 ringförmig an der Stelle des größten Durchmessers der Aufweitung 39 aufgeweitet. Dabei kann vorteilhaft eine ringförmige Dichtzone entstehen, in der die Innenwandung des Konnektionsrohrs 5 mit - vorzugsweise elastischer - Materialspannung auf dem Außenumfang des größten Außendurchmessers der Aufweitung 39 aufliegt.

Eine derartige Materialspannung durch Aufweiten kann in Verbindung mit einer hinreichenden geringen Rauhigkeit der zugeordneten Oberflächen für eine ausreichende Fluiddichtheit der Konnektion gegen die Auslegungs-Differenzdrücke zur Umgebung sorgen.

Die Innenwandung des Konnektionsrohrs 5 ist bevorzugt zylindrisch ausgeführt.

Die Innenwandung des Konnektionsrohrs 5 kann im Verhältnis zum Durchmesser des Konnektionsrohrs 5 bevorzugt dünnwandig und/oder wandstärkenkonstant ausgeführt sein. Auf diese Weise kann es vorteilhaft möglich sein, die Aufweit- und/oder Abdichtwirkung bei beliebig weitem Einschieben des Innenrohrs 1 in das Konnektionsrohr 5 konstant zu halten.

Als Werkstoff für das Konnektionsrohr 5 und/oder das Innenrohr 1 und/oder das Außenrohr 3 können, ohne darauf beschränkt zu sein, thermoplastische Kunststoffe wie Polypropylen, Polyamid, Polycarbonat, Thermoplastverbundstoffe mit PTFE (Polytetrafluorethylen) und dergleichen, eingesetzt werden.

Diese Werkstoffe können in vorteilhafter Weise preisgünstig mittels Spritzgußverfahren verarbeitet werden. Sie können zudem eine günstige Steifigkeit und/oder Zähigkeit für das vorstehend beschriebene Aufweit-Dichtprinzip aufweisen.

Typische Abmessungen des Konnektionsrohrs 5 schließen einen Innendurchmesser von 4 mm bis 8 mm, eine Wandstärke von 0,4 mm bis 1 mm sowie eine Länge von 20 mm bis 40 mm ein.

Allgemein ist ein Rohr geometriebedingt in der Nähe seiner offenen Mündung vorwiegend weniger aufweitsteif und/oder in der Nähe des angebundenen Endes vorwiegend höher aufweitsteif als in einem mittleren Bereich.

Aus diesem Grund können für die verschiedenen Konnektionsstellungen während der Herstellung der Fluidverbindung vorzugsweise jene Bereiche des Konnektionsrohrs 5 eingesetzt werden, welche etwas mehr als das Einfache des Innendurchmessers von einem oder beiden Enden des Konnektionsrohrs 5 entfernt sind.

Aufgrund ihrer vorzugsweise dünnwandigen und/oder einfachen Geometrie ist das Konnektionsrohr 5 vorzugsweise die Konnektions-Halbanordnung bzw. das System, welche(s) auf der Seite einer externen Funktionseinrichtung 400, zum Beispiel als Einmalteil, ausgeführt ist.

Die Aufweitung 39 kann wie oben beschrieben eine Dichtung in der externen Funktionseinrichtung herstellen. Die Aufweitung 39 kann ferner einen Toleranzausgleich schaffen.

Die Aufweitung 39 des Innenrohrs 1 ist bevorzugt so ausgeführt, dass zumindest der ringförmige Aufweitbereich die Gestalt einer Kugel hat. Das Zentrum der Kugel kann auf einer Mittelachse bzw. Symmetrieachse 83 des Innenrohrs 1 liegen.

Fallen die Mittel- oder die Symmetrieachse 83 des Innenrohrs 1 und die Mittel- oder die Symmetrieachse 81 des Konnektionsrohrs 5 nicht zusammen, so kann sich auf diese Weise vorteilhaft immer die gleiche ringförmige Touchier-, bzw. Abdichtgeometrie einstellen. Bei besonders hohen Ansprüchen an geringe Konnektionskräfte und/oder an besonders hohe Dichtwirkungen (etwa Dichtung dünnflüssiger Fluide, wie Flüssigkeiten und Gase) kann es möglich sein, den kugelsphärischen Ringbereich der Aufweitung 39 aus mehreren, einzelnen, schmaleren Ringdichtstegen oder aus einem Elastomer-Ringeinsatz auszuführen.

Schmale Ringdichtstege können vorteilhaft für redundante Dichtringzonen mit gleicher oder erhöhter Dichtpressung sorgen.

Ein Elastomer-Ringeinsatz kann vorteilhaft kleine Rauhigkeiten zwischen den Dichtpartnern überbrücken.

Die Aufweitung 39 des Innenrohrs 1 kann gegenüber einem Innendurchmesser des Konnektionsrohrs 5 ein Durchmesser-Übermaß aufweisen. Ein solches Durchmesser-Übermaß liegt bevorzugt in einem Bereich von 2 % bis 10 % des Innendurchmessers des Konnektionsrohrs 5.

Das Innenrohr 1 mit der Aufweitung 39 kann eine Konnektions-Halbanordnung der Behandlungsvorrichtung 300 sein.

Aufgrund von Anforderungen, wie mehrfache Verwendbarkeit und/oder Desinfizierbarkeit mit heißen Fluiden (z.B. Flüssigkeiten) weisen die Verbindungseinrichtung 100 bzw. das Innenrohr 1 und/oder die Aufweitung 39 des Innenrohrs 1 bevorzugt Werkstoffe wie rostfreien Stahl, Titan, Stähle mit keramischen, oxidischen und/oder PTFE-(Polytetrafluorethylen-) haltigen Beschichtungen und/oder spritzgießbare Sinterkeramiken wie Zirkonoxid auf oder sind aus solchen gefertigt.

Es ist jedoch ebenso möglich, die erfindungsgemäße Verbindungseinrichtung 100 als Einmalteil, z.B. mit thermoplastischen Kunststoffen, auszuführen.

Insbesondere bei Verwendung von Aufweitungen 39 mit dünnen oder schmalen Ringdichtstegen und/oder mit einem Elastomer-Ringeinsatz (Überlagerung mit Negativ-Aufweitung) kann es möglich sein, die Konnektions-Halbanordnung umkehrt auszuführen, d.h. das Konnektionsrohr 5 als steife bzw. formfeste Komponente der Behandlungsvorrichtung 300 oder als steife bzw. formfeste Komponente der externen Funktionseinrichtung 400 ausführen. Dies ist insbesondere dann bevorzugt, wenn die Gestaltung dünnwandiger Konnektionsrohre 5 nicht oder nur mit großem Aufwand oder Kosten möglich ist.

Das Außenrohr 3 weist einen Redundanzdichtwulst 91 als Beispiel einer weiteren, redundanten Dichtsicherung auf. Beim Erreichen einer Konnektionsposition wird i.d.R. ein Kontakt zwischen der Außenwandung des Konnektionsrohrs 5 und der Innenwandung 15 des Außenrohrs 3 hergestellt. Der Redundanzdichtwulst 91 kann dazu dienen, das Konnektionsrohr 5 radial abzustützen.

Der Redundanzdichtwulst 91 hat vorwiegend zwei Aufgaben: Erstens dient der Redundanzdichtwulst 91 der radialen Abstützung des Konnektionsrohrs 5 zur Aufrechterhaltung der erfindungsgemäßen Aufweit-Hauptabdichtung. Zweitens dient der Redundanzdichtwulst 91 als redundante sekundäre Abdichtung. Der in Fig. 12 links neben dem Redundanzdichtwulst 91 gezeigte O-Ring dient als Feder.

Der Redundanzdichtwulst 91 kann ringförmig ausgestaltet sein.

Fig. 12 zeigt eine typische Konnektionsstellung der erfindungsgemäßen Verbindungseinrichtung für einen Redundanzdichtwulst 91.

Das Konnektionsrohr 5 der Konnektorbaugruppe 200 wird in dieser Stellung in einer Ringzone an der Innenwandung 15 des Außenrohrs 3 wiederum gegen die Verbindungseinrichtung 100 gedrückt. Es kann eine fluidische Abdichtung vorliegen.

Der Innendurchmesser des am Außenrohr 3 angebrachten Redundanzdichtwulsts 91 kann etwa 2 % bis 10% kleiner gewählt sein als der Außendurchmesser des im Wesentlichen zylindrischen und/oder glattwandigen Konnektionsrohrs 5.

Wenigstens einer der beiden Dichtpartner, das Außenrohr 3 und/oder das Konnektionsrohr 5, in der Regel beide, können vorzugsweise elastisch ausgebildet sein.

Durch das im Wesentlichen axiale Ineinanderschieben des Konnektionsrohrs 5 in das Außenrohr 3 kann es im Bereich des Redundanzdichtwulsts 91 zu einer Kompression radial nach innen auf das Konnektionsrohr 5 bzw. zu einer Aufweitung des Redundanzdichtwulsts 91 radial nach außen kommen.

Wie bei der Hauptabdichtung kann eine ringförmige Zone entstehen, in der das Konnektionsrohr 5 mit radialer Spannung gegen den Redundanzdichtwulst 91 verpresst wird.

Auf diese Weise kann wiederum die Materialspannung in Verbindung mit einer ausreichenden Glattheit bzw. mit einer ausreichenden Angleichung der zugeordneten Oberflächen vorteilhaft zu einer Fluiddichtheit des Konnektionsrohrs 5 gegenüber dem Außenrohr 3 führen.

Der Redundanzdichtwulst 91 ist vorzugsweise in unmittelbarer Nachbarschaft zur Aufweitung 39 der Hauptabdichtung mit einem axialem Versatz von bis zum etwa dem Einfachen des Innendurchmessers des Konnektionsrohrs 5 in Richtung der offenen Rohrmündung des Konnektionsrohrs 5 angeordnet.

Auf diese Weise kann vorteilhaft einer Ermüdung der Aufweit-Hauptabdichtung durch die Aufweitung 39 durch plastische Verformung des Konnektionsrohrs 5 entgegengewirkt werden.

Durch die versetzte Anordnung der Aufweitung 39 kann einerseits für eine verbleibende elastische Verformungsmöglichkeit des Konnektionsrohrs 5 nach außen wie nach innen gesorgt werden. Andererseits kann ein Rohr allgemein bei Aufweitung dazu neigen, sich zum offenen Rohrende hin trichterförmig aufzuweiten. Dieser Art der Aufweitung wirkt die versetzte Anordnung der Redundanz-Abdichtringzone des Redundanzdichtwulsts 91 ebenfalls vorteilhaft entgegen.

Der Redundanzdichtwulst 91 kann - wie unabhängig hiervon auch die Aufweitung 39 - aus harten Werkstoffen wie etwa Metallen und/oder Keramik, aus nachgiebigen Werkstoffen wie PTFE und/oder POM (Polyoxymethylen), und/oder aus elastomeren Werkstoffen wie z. B. Siliconkautschuk und dergleichen hergestellt sein oder solche umfassen.

Besonders bevorzugt ist der in Fig. 12 dargestellte Redundanzdichtwulst 91 aus PTFE in einem Stück mit dem Kopfkörper 57 des Außenrohrs 3 hergestellt.

Das PTFE-Material kann durch eine Vergütung (Kaltrecken) und/oder durch eine Lagerung gegen einen elastischen Ring aus Elastomeren und/oder mittels ringförmiger Federn eine bleibende Elastizität radial nach innen erhalten.

Durch das bevorzugte Material PTFE mit geringem Reibbeiwert kann die zur Abdichtung erforderliche axiale Schiebekraft vorteilhaft minimiert werden.

Die elastischen Elemente können vorteilhaft eine konstante Dichtwirkung auch bei einer zulässigen Abnützung der PTFE-Schicht bewirken.

Mit der erfindungsgemäßen Verbindungseinrichtung 100 kann in vorteilhafter Weise eine Dichtigkeitsüberwachung und/oder ein Leckageschutz erreicht werden.

In der Konnektionsstellung der Figur 12 ist das Konnektionsrohr 5 innen mit dem Fluidanschluss 51 des Innenrohrs 1 fluiddicht verbunden. Das Konnektionsrohr 5 ist ferner fluiddicht gegenüber dem Fluidanschluss 53 des Außenrohrs 3.

Zur Überwachung der Dichtheit kann das folgende Verfahren bevorzugt angewandt werden: Das Innenlumen 33 des Innenrohrs 1 dient bevorzugt der regulären Fluidkonnektion. Das Außenlumen 37 des Innenrohrs 1 ist bei bzw. während der Konnektion wahlweise gas-, beispielsweise luft-, und/oder flüssigkeitsgefüllt. Das Außenlumen 37 ist bevorzugt über ein Druckbegrenzungsventil gegen eine Leitung zum Abwasserzweig, z.B. einen Tropfwasserablauf (in Fig. 12 nicht gezeigt) geschaltet.

Der Ansprechdruck des Druckbegrenzungsventils kann geringer eingestellt sein als der in der inneren Fluidverbindung maximal auftretende Flüssigkeitsdruck. Der Ansprechdruck des Druckbegrenzungsventils kann weiterhin kleiner als der minimal mit dem Redundanzdichtwulst 91 erzielbare Dichtheitsdruck zur Umgebung sein.

Besonders bevorzugt liegt der Ansprechdruck des Druckbegrenzungsventils sogar unter dem Umgebungsdruck.

Bei auftretender Undichtigkeit kann sich schließlich das Druckbegrenzungsventil öffnen und die Leckageflüssigkeit kontrolliert in den Abwasserzweig übertreten lassen. Auf diese Weise kann vorteilhaft der Schaltzustand des Druckbegrenzungsventils abgefragt werden und somit eine eventuelle Undichtheit der Hauptdichtung bereits im Rahmen der Selbstüberprüfungsroutine vor Beginn des relevanten Behandlungsverfahrens erkannt werden. Auf diese Weise kann es wiederum vorteilhaft möglich sein, frühzeitig Gegenmaßnahmen, wie ein Auswechseln der externen Funktionseinrichtung 400 und/oder ein Auswechseln verschlissener Komponenten der Behandlungsvorrichtung 300, zu ergreifen.

Alternativ zur Schaltzustandsabfrage des Druckbegrenzungsventils kann auch das Signal eines zusätzlich vorgesehenen Drucksensors ausgewertet werden.

Alternativ kann es auch möglich sein, an den Fluidanschluss 53 des Außenrohrs 3 ein Vakuum anzulegen (z.B. bei Verwendung einer Fluidverbindung unter Unterdruckbedingungen) und/oder eine direkte Verbindung zum zur Atmosphäre offenen Abfluss (z.B. bei Verwenden einer Fluidverbindung unter Überdruckbedingungen) herzustellen und im Rahmen der Selbstüberprüfungsroutine einen Druckhaltetest der inneren Fluidkonnektion - insbesondere zwischen Innenrohr 1 und Konnektionsrohr 5-durchzuführen.

Das zuvor als erstes beschriebene Überwachungsverfahren kann vorteilhafter Weise bereits vor Beginn der eigentlichen Behandlung bzw. Hauptbehandlung, wie beispielsweise einer Dialysebehandlung, durchgeführt werden. Es kann in vorteilhafter Weise kontinuierlich auch während der Hauptbehandlung wirken.

Das zuvor als zweites beschriebene Überwachungsverfahren kann sich auf eine Dichtheitsüberwachung vor Beginn der Hauptbehandlung beschränken.

Beide Fluidanordnungen können in vorteilhafter Weise gewährleisten, dass zu keinem Zeitpunkt Behandlungsfluide in die Umgebung austreten. Stattdessen werden eventuelle Leckagefluide kontrolliert in den Abwasserzweig der Behandlungsvorrichtung 300 abgeführt.

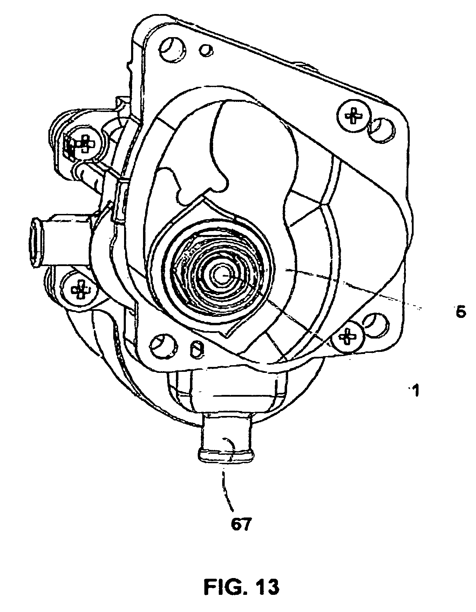

**Fig. 13** zeigt eine Ansicht auf eine erfindungsgemäße Verbindungseinrichtung 100 in einer Konnektionsposition.

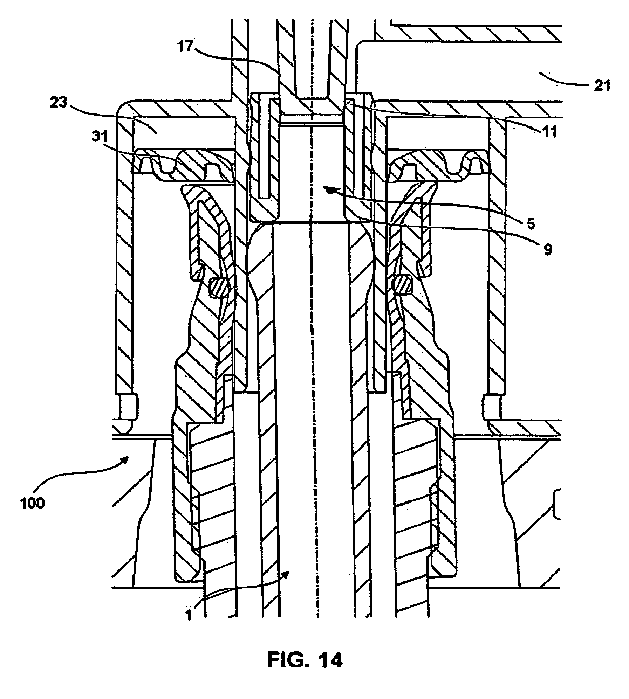

**Fig. 14** zeigt die erfindungsgemäße Verbindungseinrichtung 100 im Längsschnitt in einer verschlossenen Stellung.

Es ist eine Fluidkommunikation zwischen dem Innenrohr 1 und dem Konnektionsrohr 5 hergestellt.

Der Aufnahmeraum 23 ist, z.B. im Vergleich zu demjenigen in Fig. 12, durch Hochschieben der Berührschutzscheibe 31 weiter verkleinert.

Die Verschlusshülse 9 ist von unten (bezogen auf die Anordnung in Fig. 14) durch den Verschlusskragen 11 in den Verschlusszapfen 17 gedrückt.

Die Verschlusshülse 9 wird durch Verfahren oder Verschieben der Verbindungseinrichtung 100 in die Verschlussposition (bei offener Spülkappe) in Fig. 14 nach oben verfahren. Die Verschlusshülse 9 verschießt den Kanal 21 zur externen Funktionseinrichtung.

Das Verschließen der externen Funktionseinrichtung kann beispielsweise am Ende einer Behandlungssitzung erfolgen.

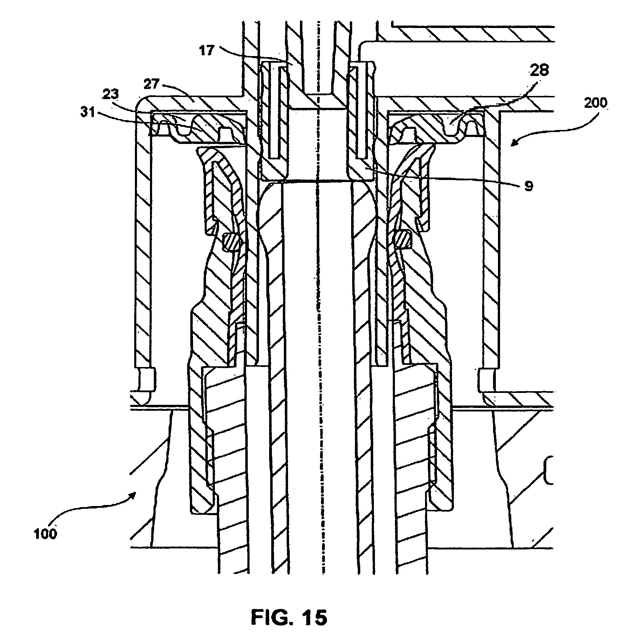

**Fig. 15** zeigt die erfindungsgemäße Verbindungseinrichtung 100 im Schnitt in einer maximal zulässigen Verschiebung der Verschlussstellung.

Zwischen der Berührschutzscheibe 31 und der Innenseite der Außenwandung 27 verbleibt vorzugsweise ein Restspalt 28. Dies kann vorteilhaft zu einem sicheren und störungsfreien Betrieb beitragen. Der Aufnahmeraum 23 nimmt eine minimale Größe ein.

Die Verschlusshülse 9 ist wiederum weiter in den Verschlusszapfen 17 gedrückt.

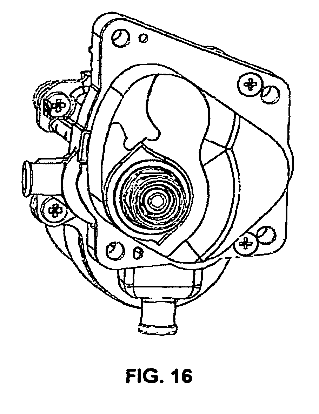

**Fig. 16** zeigt eine Ansicht auf eine erfindungsgemäße Verbindungseinrichtung 100 ohne Spülkappe.

Um beispielsweise ein Reinigungsprogramm durchzuführen, kann die Verbindungseinrichtung 100 aus der Verschlussposition in eine Spülposition gebracht werden.

Zum Verfahren der Spülkappe in Spülposition wird die Supporteinrichtung bzw. der Support so weit axial entgegen der Konnektionsrichtung verfahren, dass die Spülkappe axial fluchtend über die Verbindungseinrichtung verschwenkt werden kann. Nach erfolgter Schwenkbewegung verfährt der Support bzw. die Supporteinrichtung in Konnektionsrichtung bis zur dichtenden Verbindung der Verbindungseinrichtung mit der Spülkappe.

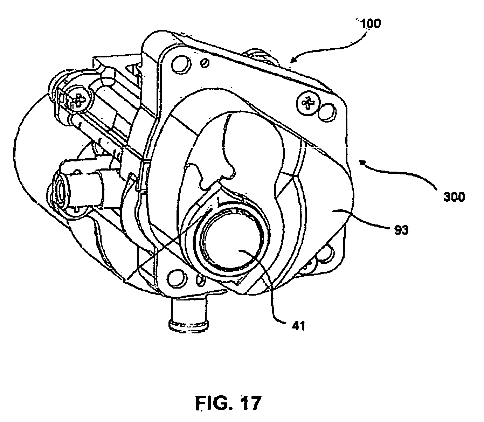

**Fig. 17** zeigt eine Ansicht einer erfindungsgemäßen Verbindungseinrichtung 100 in einer Spülposition. Das Gehäusevorderteil 93 der Behandlungsvorrichtung 300 verdeckt hinter ihr liegende Komponenten der Verbindungseinrichtung 100 und/oder der Behandlungsvorrichtung 300. Bezugszeichen verdeckter Elemente sind in der folgenden Beschreibung angegeben, um ein Verständnis des Zusammenwirkens derselben zu erleichtern.

Die Spülposition kann der Grundstellung der Verbindungseinrichtung 100 entsprechen.

In der Spülposition ist die Spülkappe 41 vor die Öffnung (der Verbindungseinrichtung 100) im Gehäusevorderteil 93 der Behandlungsvorrichtung 300 geschwenkt. Vorzugsweise schließt die Spülkappe 41 axial bündig mit der Vorderseite einer Aktor-Sensor-Matte (nicht gezeigt) ab. Ein möglicher Ringspalt zwischen der Spülkappe 41 und dem Gehäusevorderteil 93 der Behandlungsvorrichtung 300 kann beispielsweise mit einem O-Ring gegen eindringende Fluide, insbesondere eindringende Flüssigkeiten, abgedichtet werden.

Die Verbindungseinrichtung 100 taucht vorzugsweise derart in die Spülkappe 41 ein, dass der Dichtkörper 59 des Außenrohrs 3 bzw. eine Außenkante desselben in der Spülkappe 41 gegen ein Äußeres abdichtet ist.

Wenn der Dichtkörper des Außenrohrs 3 aus einem PTFE-Verbundstoff gefertigt ist, kann er den weicheren Partner dieser Dichtpaarung darstellen. Er kann deshalb vorzugsweise so gestaltet sein, dass er vorteilhaft ohne Öffnen der Behandlungsvorrichtung 300 ausgetauscht werden kann.

Zum Reinigen der Verbindungseinrichtung 100 wird allgemein Reinigungsflüssigkeit durch das Innenrohr 1 zu- und durch das Außenrohr 3 abgeführt.

Ein Hallsensor, z.B. ein H2Conn (S502), kann den auf dem Schwenkhebel 75 angebrachten Magneten 77 detektieren. Auf diese Weise kann die axiale Position einer Spindelmutter (und damit des Konnektionsrohrs 5 der externen Funktionseinrichtung 400) mit Hilfe des Potentiometers, z.B. des Linearpotentiometers SPosConn (S503), ermittelt werden. Die axiale Position der Spülkappe 41 kann aus dem Abstand der Außenkante des Dichtkörpers vom Gehäusevorderteil 93 der Behandlungsvorrichtung 300 ermittelt werden.

Um aus der Spülstellung in die Konnektionsstellung überzugehen, kann folgender Bewegungsablauf ausgeführt werden: Zunächst erfolgt eine axiale Bewegung der Verbindungseinrichtung 100 mitsamt der Spülkappe 41 entgegen der Konnektionsrichtung (in der Fig. 17 nach links). Anschließend wird die Verbindungseinrichtung 100 in axialer Richtung aus der Spülkappe 41 heraus bewegt. Die Spülkappe 41 kann stehen bleiben. Sodann kann eine Schwenkbewegung der Spülkappe 41 so weit erfolgen, dass eine freie Bewegung der Verbindungseinrichtung 100 in Konnektionsrichtung möglich wird. Zuletzt kann die Verbindungseinrichtung 100 in axialer Richtung aus der Frontebene der Behandlungsvorrichtung 300 heraus und in die externe Funktionseinrichtung 400 hinein bewegt werden.

Um aus der Spülposition in die Konnektionsposition zu gelangen, kann die Verbindungseinrichtung 100 axial in Konnektionsrichtung verfahren werden.

Durch das Aufschwenken des Schwenkhebels 75 gibt die Spülkappe 41 vorzugsweise eine Öffnung im Gehäusevorderteil 93 frei. Die Verbindungseinrichtung 100 kann nach vorn verfahren werden. Die Verbindungseinrichtung 100 kann nun in das Konnektionsrohr 5 der Konnektorbaugruppe 200 der externen Funktionseinrichtung 400 eindringen.

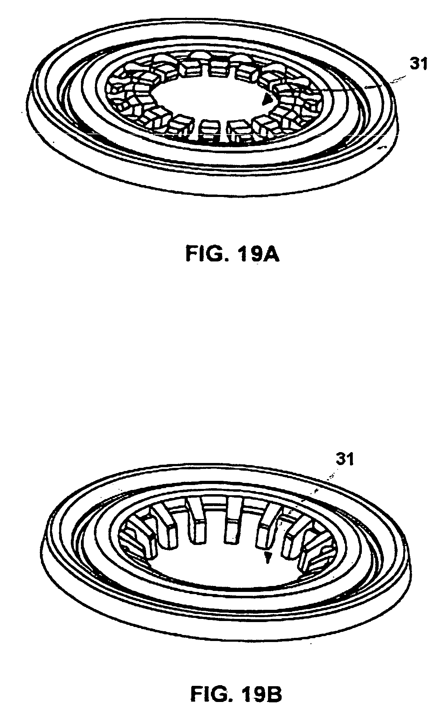

Im Folgenden werden bevorzugte Ausführungsformen der erfindungsgemäßen Verbindungseinrichtung 100 bzw. einzelner Komponenten derselben und einzelne Merkmale, welche ungeachtet anderer Merkmale in jeder erfindungsgemäßen Ausführungsform Verwendung finden können soweit vom Fachmann als sinnvoll erachtet, beschrieben, insbesondere unter Bezugnahme auf die **Fig. 19** bzw. **Fig. 19A** bis **Fig. 28****.**

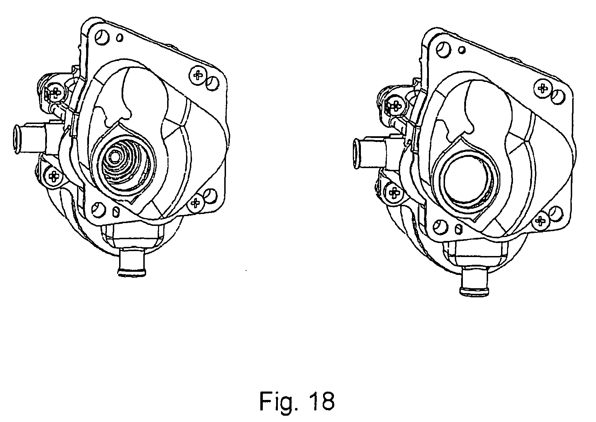

Zur Erleichterung des Verständnisses der beschriebenen Ausführungsformen werden Bezugszeichen angegeben, deren allgemeine Beschreibung bzw. Zuordnung vorwiegend den Fig. 1 bis 18 entnehmbar ist.

In einer weiteren bevorzugten Ausführungsform stellt die vorliegende Erfindung eine besondere laterale Beweglichkeit und/oder Vorzentrierung bereit.

Externe Funktionseinrichtungen 400, z.B. Disposablekassetten, können eine Vielzahl gleichzeitig räumlich auszurichtender funktioneller Einrichtungen aufweisen. Oftmals ist vorgesehen, diese funktionellen Einrichtungen, z.B. Konnektoren, Sensoren, Aktoren und dergleichen, zu oder an entsprechenden funktionellen Einrichtungen und/oder Anordnungen von Behandlungsvorrichtungen 300 auszurichten.

Eine Grundausrichtung und/oder Fixierung der gesamten externen Funktionseinrichtung 400 kann in der Regel durch eine separate Einrichtung erfolgen. Typischerweise erfolgt eine zentrierende Verrastung der externen Funktionseinrichtung 400 zum Chassis 47 der Behandlungsvorrichtung 300 und/oder eine Fixierung der externen Funktionseinrichtung 400 durch Verpressung und/oder formschlüssigen Einbau zwischen dem Chassis 47 der Behandlungsvorrichtung 300. Optional können dabei eine Gummiplatte 49 und/oder eine Türplatte als Abdeckungseinrichtung 85 eingesetzt werden (siehe z.B. Fig. 3).

Wenn zwei externe Funktionseinrichtungen 400 miteinander in Fluidverbindung gebracht werden sollen, kann die Ausrichtung zwischen der Verbindungseinrichtung 100 und der Konnektorbaugruppe 200 bzw. zwischen einer Verbindungseinrichtung 100 einer ersten externen Funktionseinrichtung 400 und einer Konnektorbaugruppe 200 einer zweiten externen Funktionseinrichtung 400 direkt mittels des Innenrohrs 1 und des Außenrohrs 3 der Verbindungseinrichtung 100 und des Konnektionsrohrs 5 der Konnektorbaugruppe 200 erfolgen.

Eine Verbindung der Verbindungseinrichtung 100 und/oder der Konnektorbaugruppe 200 mit einem Anschluss bzw. Port einer Behandlungsvorrichtung 300 kann beispielsweise über ein Gewinde und/oder einen Bayonettverschluss und/oder über ein Schwenkjoch (wie z.B. beim Rinse-Port der aktuellen 5008-Dialysemaschine der Fa. Fresenius Medical Care. Bad Homburg, Deutschland) erfolgen.

Bislang stellte sich z.B. das Problem, dass es bei Ungenauigkeiten der Einzelkomponenten und/oder bei der Montage der Einzelkomponenten zu Baugruppen allgemein zu Abweichungen der Teilungsabstände der Rohre (Innenrohr 1, Außenrohr 3, Konnektionsrohr 5) zwischen den Systemen bzw. Konnektionssystemen kommen konnte.

Aufgrund der Grundausrichtung und/oder Fixierung einer gesamten externen Funktionseinrichtung 400 gegen eine gesamte Behandlungsvorrichtung 300 und/oder zweite externe Funktionseinrichtung 400 durch eine übergeordnete Einrichtung, beispielsweise eine Verpresseinrichtung, konnte es im Stand der Technik zusätzlich zu einem (weiteren) toleranzbedingten oder fertigungsbedingten lateralen Versatz der Konnektionssysteme kommen.

In einem solchen Fall konnten Fluidkonnektionen nur dann optimal erfolgen, wenn sowohl die Verbindungseinrichtung 100 als auch die Konnektorbaugruppe 200 mit hinreichend geringer lateraler Abweichung der jeweils zugeordneten Konnektionsachsen (z.B. die Mittelachsen 81,83) in die Konnektionsstellungen überführt werden konnten.

Bei einer typischen herkömmlichen Anordnung mit mehreren Konnektionsrohren 5 externer Funktionseinrichtungen 400 kann die maximal zulässige laterale Positionsabweichung einer Konnektionsmündung, z.B. des Innenrohrs 1, gegen z.B. die Konnektionsmündung des Konnektionsrohrs 5 z.B. etwa +/- 0,2 mm betragen.

Bis zu einer solchen Positionsabweichung könnten sich die Verbindungseinrichtung 100 und die Konnektorbaugruppe 200, insbesondere das Konnektionsrohr 5 der Konnektorbaugruppe 200, noch unter hinreichend geringer Lateralkraft von z.B. etwa kleiner als 10 N durch Verbiegung in Konnektionsstellung zueinander zentrieren, ohne dass Undichtheit hervorgerufen würde.

In der Realität einer der oben genannten Multi-Konnektor-Anordnungen können jedoch laterale Abweichungen der zugeordneten Konnektor-Mündungspositionen nach der Grundausrichtung und Fixierung von etwa bis zu +/-1,5 mm auftreten.

Damit die einander zugeordneten Rohre (Innenrohr 1, Konnektionsrohr 5 und Außenrohr 3) erfolgreich ineinander geschoben werden können, benötigen sie vorzugsweise entsprechend ausgeführte Einführtrichter 87 in Verbindung mit einer ebenso großen lateralen Beweglichkeit mindestens jeweils einer bei beiden Konnektionspartner, also der Verbindungseinrichtung 100 und/oder der Konnektorbaugruppe 200, relativ zum anderen.

Diese laterale Beweglichkeit kann bevorzugt durch eine Schwenklagerung 455 der erfindungsgemäßen Verbindungseinrichtung 100 relativ zum Chassis 47 der Behandlungsvorrichtung 300 gewährleistet werden.

Die dargestellte Lagerungsart führt vorteilhaft zu einer hinreichenden lateralen Beweglichkeit der Konnektionsmündung des Innenrohrs 1 der erfindungsgemäßen Verbindungseinrichtung 100 relativ zum Chassis 47 der Behandlungsvorrichtung 300.

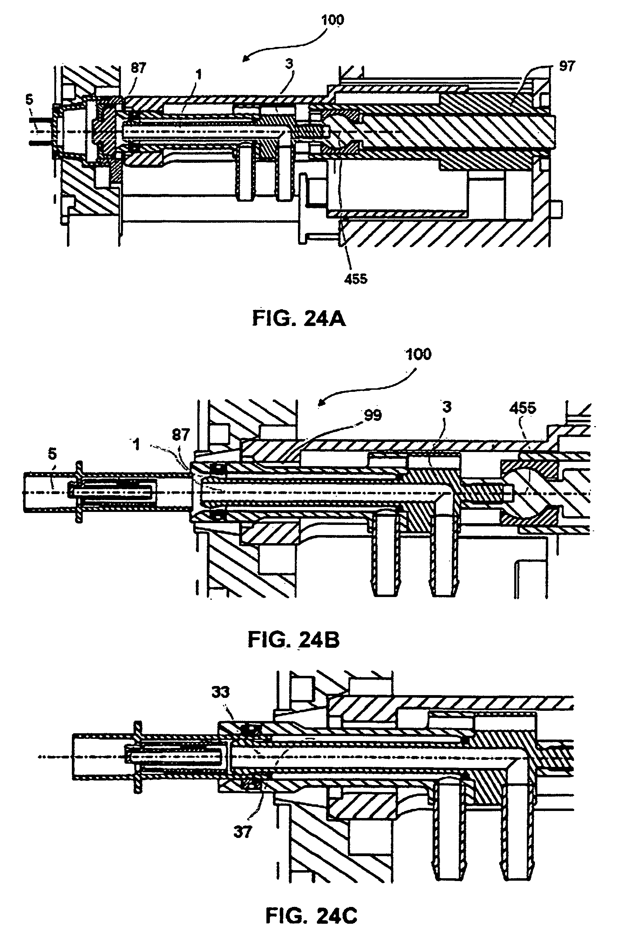

Das Außenrohr 3 weist mit hinreichender Entfernung zur Konnektionsmündung des Innenrohrs 1 (vorzugsweise etwa 5-mal bis 20-mal den Durchmesser des Konnektionsrohrs 5) eine kugelige Gestalt auf, welche in entsprechenden Halbkugelpfannen, dem Oberteil 451 und dem Unterteil 453 der Supporteinrichtung 45, drehbar und/oder spielarm gelagert ist (siehe Fig. 3 sowie Fig. 24A-C).

Zum Sichern einer lateralen Beweglichkeit können selbstverständlich auch andere Konstruktionsmöglichkeiten eingesetzt werden.

Da die erfindungsgemäße Verbindungseinrichtung 100 auch in horizontaler Lage unter Schwerkrafteinfluss Verwendung finden kann, und da die laterale Beweglichkeitseinrichtung üblicherweise reibungsbehaftet und deshalb nicht selbsttätig rückstellend ist, wird erfindungemäß vorzugsweise eine Vorzentriereinrichtung eingesetzt.

Die Vorzentriereinrichtung richtet vorzugsweise mindestens eine der erfindungsgemäßen Verbindungseinrichtung 100 und/oder der Konnektorbaugruppe 200 vor einer erneuten Konnektion in einer Toleranzmitte aus.

Diese Vorzentriereinrichtungen können bevorzugt gewährleisten, dass eine maximale seitliche Kraft zwischen der erfindungsgemäßen Verbindungseinrichtung 100 und der Konnektorbaugruppe 200 während der Konnektion auf einen vorbestimmten Wert, beispielsweise etwas kleiner als etwa 10 N, begrenzt wird.

Die Vorzentrierungseinrichtung ist insbesondere von Vorteil, wenn die erfindungsgemäße Verbindungseinrichtung 100 und die Konnektorbaugruppe 200 aufgrund ihrer lateralen Beweglichkeit bereits vor der Konnektion eine zu ausgeprägt lateral abweichende Lage einnehmen könnten. Bei einem solchen Fall könnte dann ein entsprechend größerer Einführtrichter 87 erforderlich sein, was allgemein jedoch unerwünscht wäre, da damit die Baugröße der Anordnung der erfindungsgemäßen Verbindungseinrichtung 100 ungünstig anwachsen würde.

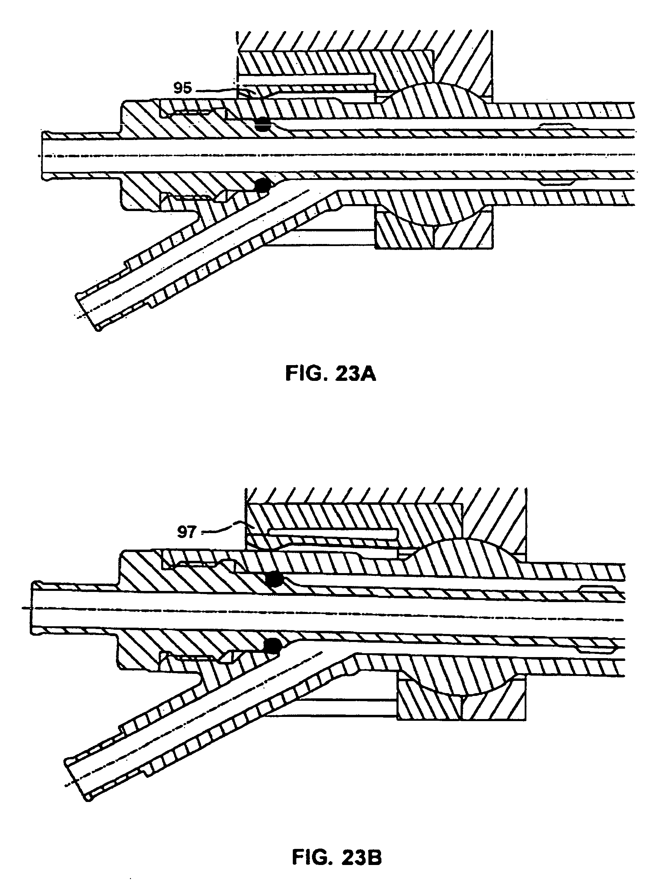

Die Figuren 23A und 23B zeigen eine Ausführung der Vorzentrierung über federnd ausienkbare Zungen oder Vorzentrier-Federzungen 95 (vorzugsweise drei Zungen).

Die Vorzentrier-Federzungen 95 können die Verbindungseinrichtung 100 in eine Nullstellung bzw. Grundstellung bringen und gleichzeitig selbst bei maximaler Auslenkung eine hinreichend geringe laterale Kraft ausüben.

Vorzentrier-Anschläge 97 können eine Begrenzung der Lateralauslenkung auf den maximal zulässigen Wert (ggf. in Abhängigkeit von den Abmaßen der Anordnung des Einführtrichters 87) sicherstellen.

Die Figuren 24A bis 24C zeigen eine alternative Anordnung, bei welcher die Schwenklagerung 455 bei horizontaler Ausrichtung als ausbalancierte Waage ausgeführt ist. Von der Grundstellung der Fig. 24A bis in eine Zwischenstellung der Fig. 24B wird das Außenrohr 3 durch eine Vorzentrier-Röhre 99 geführt, bis eine Mündung des Innenrohrs 1 der erfindungsgemäßen Verbindungseinrichtung 100 mit dem Einführtrichter 87 eine Überlappung zur Mündung des Konnektionsrohrs 5 der Konnektorbaugruppe 200 aufweist.

Von dieser Stellung an bis zu Konnektionsstellung nach Fig. 24C und darüber hinaus kommt die Vorzentrier-Röhre 99 außer Eingriff, so dass keine rückstellenden Federkräfte wie bei bisher bekannten Anordnungen lateral auf die Konnektionspartner einwirken.

Bevorzugt sind die fluidischen Anschluss-Leitungen des Innenlumens 33 des Innenrohrs 1 sowie des Außenlumens 37 des Innenrohrs 1 als leicht bewegliche und leicht biegbare Schlauchleitungen, besonders bevorzugt aus Silikongummi, ausgeführt.

Dabei ist eine Anordnung derselben möglichst nahe an einem Gelenk-Mittelpunkt der Schwenklagerung 455 vorzuziehen. Zur Reduktion der lateralen Auslenkkräfte auf die Verbindungseinrichtung 100 kann es besonders vorteilhaft sein, den Schlauchleitungen oder Abschnitten hiervon eine spiralige Form zu geben.

Weiterhin kann es besonders vorteilhaft sein, die Schlauchleitungen so auszurichten und/oder zu fixieren, dass die lateralen schlauchanschlussbedingten Auslenkkräfte in der mittigen Vorzentrierstellung annähernd gleich Null sind.

In einer extremen Auslenkstellung der Schwenklagerung 455 sollten vorzugsweise alle auf die Konnektion einwirkenden lateralen Kräfte kleiner sein als die maximal zulässige Lateralkraft.

In einer weiteren bevorzugten Ausführungsform stellt die vorliegende Erfindung eine laterale, axiale und/oder winklige Feinausrichtung und/oder einen Toleranzausgleich bereit.

Die relevanten Vorgänge des Toleranzausgleichs werden im Folgenden im Rahmen einer Darstellung der Bewegungsvorgänge während der Konnektion erläutert.

Von einer Grundstellung der Verbindungseinrichtung 100 (z.B. nach Fig. 4 oder Fig, 24A) oder von einer Ankoppelstellung (etwa nach Fig. 7) bis in eine Stellung nach Fig. 24B ist die zuvor beschriebene Vorzentrierung wirksam.

Ab dieser Stellung nach Fig. 24B ist das Konnektionsrohr 5 minimal in den Einführtrichter 87 des Außenrohrs 3 eingedrungen, so dass der Einführtrichter 87 die wesentlichen Aufgaben der weiteren Zentrierung der Verbindungseinrichtung 100 bzw. des Innenrohrs 1 und der Konnektorbaugruppe 200 bzw. des Konnektionsrohrs 5 erfüllen kann.

Bei einer weiteren Konnektionsbewegung kommt es ab der Stellung nach Fig. 8 zur Berührung der Außenkontur bzw. einer Stirnfläche der Berührschutzscheibe 31 des Konnektionsrohrs 5 mit einer Stelle des Einführtrichters 87 und damit zur weiteren lateralen Zentrierung bis zur Stellung nach Fig. 9, welche den Abschluss der Trichter-Zentrierbewegung darstellt.

Den letzten Part der lateralen Zentrierung kann die Aufweitung 39 des Innenrohrs 1 nach erstmaliger Berührung mit der verrundeten Mantelfläche der Innenwandung 7 des Konnektionsrohrs 5 übernehmen, siehe beispielsweise die Fig. 10.

Von dieser Stellung an für alle weiteren Einführwege der Verbindungseinrichtung 100 und der Konnektorbaugruppe 200 ist die Feinzentrierung abgeschlossen. Die bevorzugt kugelsphärische Gestaltung der Aufweitung 39 kann in Verbindung mit dem Außendurchmesser des Innenrohrs 1 und in Verbindung mit dem im Verhältnis dazu etwa 5 % bis 30 % größeren Innendurchmesser des Konnektionsrohrs 5 dazu geeignet sein, den vollständigen lateralen Toleranzausgleich über die zuvor beschriebene Schwenkbewegung der Behandlungsvorrichtung 300 zu erlauben. Der dabei entstehende maximale Winkelversatz zwischen den Konnektorachsen bzw. Mittelachsen 81, 83 führt vorteilhaft nicht zu einer mechanischen Kollision oder zu einer Undichtheit der Hauptabdichtung und/oder der Redundanzabdichtung.

In einer bevorzugten Ausführung beträgt die Toleranz des Axialversatzes zwischen der erfindungsgemäßen Verbindungseinrichtung 100 und der Konnektorbaugruppe 200 etwa +/- 1,5 mm. Dieses Toleranzmaß kann zu verringertem Herstellaufwand der beteiligten Strukturen führen.

Auch in Bezug auf die axiale Ausrichtung zwischen den beiden Konnektionssystemen kann es insbesondere bei maschinell betätigten Mehrfachkonnektionen - in externen Funktionseinrichtungen 400, z.B. Kassettenanordnungen, zu deutlichen Toleranzen kommen.

Die Anforderung nach hoher Verträglichkeit gegen axiale Toleranzen kann sich noch weiter erhöhen, wenn angestrebt wird, dass die Bestandteile des Konnektionssystems der Behandlungsvorrichtung 400 inklusive des Antriebs bzw. der Antriebseinheit nach Möglichkeit ohne individuelle Kalibrierung zusammenbau- und/oder auswechselbar sein sollen.

Weiterhin ist es erstrebenswert, dass die Antriebe der Behandlungsvorrichtung 400 möglichst einfache und preisgünstige Referenziereinrichtungen, Wegmesseinrichtungen und/oder Antriebseinheiten aufweisen.

So kann es beispielsweise genügen, eine absolute Referenzposition für den Linearantrieb über eine Lichtschranke darzustellen und/oder die gefahrenen Wege alleine über das Auszählen von Schrittmotorimpulsen zu ermitteln.

Im vorliegenden Fall können die Elemente der beiden Konnektionssysteme so ausgelegt sein, dass eine axiale Toleranz von etwa +/- 1,5 mm für alle relevanten Stellungen der Konnektoranordnung zulässig ist.

Die erfindungsgemäße axiale Toleranzverträglichkeit kann sich z. T. auch daraus ergeben, dass es sich bei der Hauptabdichtung und/oder bei der Redundanzabdichtung um radiale Dichtungen im Zusammenspiel mit im Wesentlichen zylindrischen Konnektionsrohren 5 handelt. Im Detail veranschaulichen die Fig. 12, 14 und 15, wie in allen relevanten Konnektionspositionen sowohl eine sichere Konnektion als auch eine sichere Offen- oder Verschlussstellung der Konnektorbaugruppe 200 einer externen Funktionseinrichtung 400 gewährleistet werden kann.

In einer weiteren bevorzugten Ausführungsform stellt die vorliegende Erfindung einen optimierten Berühr- und/oder Hustschutz der Konnektorbaugruppe 200 einer externen Funktionseinrichtung 400 bereit. Der optimierte Berühr- und/oder Hustschutz kann insbesondere für den hygienisch kritischen fluidführenden Innenbereich des Konnektionsrohrs 5 vorteilhaft sein.

Die vorliegende Erfindung ermöglicht ferner eine verbesserte Sterilisierbarkeit, insbesondere eine verbesserte Gassterilisierbarkeit.

Üblicherweise umfassen bisher bekannte übliche Konnektoren externer Funktionseinrichtungen 400 eine Verschlusskappe oder eine Abziehfolie, welche vor Beginn der Konnektion manuell entfernt werden muss.

Nach Abnahme dieser Schutzeinrichtungen ist die Mündung des herkömmlichen Konnektors bzw. der herkömmlichen Verbindungseinrichtung nicht mehr vor dem Zutritt von Fremdstoffen durch Luftübertragung, etwa durch Husten oder Niesen, geschützt.

Um dem entgegen zu wirken, ist das Konnektionsrohr 5 der externen Funktionseinrichtung 400 i.d.R. bekanntermaßen mit einer weiteren äußeren Röhrenstruktur ausgestattet, gegenüber der das eigentliche Konnektionsrohr 5 so weit zurücksteht, dass es bei versehentlicher Berührung nicht zu einer direkten Berührung zwischen einem menschlichen Finger und der Stirnfläche des Konnektionsrohrs 5 kommen kann.

Einen neuen Lösungsansatz der vorliegenden Erfindung stellt die Berührschutzscheibe 31 (in den Fig. 19A und 19B) in den Stellungen nach den Fig. 7 bis 12 sowie 14 und 15 dar. Die Berührschutzscheibe 31 wird, wie in den Figuren gezeigt, mittels einer Rastanordnung 29 (z.B. Fig. 2) von ihrer Montage bis zum Einsetzen der externen Funktionseinrichtung 400 in die Behandlungsvorrichtung 300 an der externen Funktionseinrichtung 400 gehalten.

Die Berührschutzscheibe 31 deckt mit segmentierten und/oder biegbaren Segmenten die Stirnfläche des Konnektionsrohrs 5 ab. Dadurch kann die Mündung des Konnektionsrohrs 5 vorteilhaft nicht berührt und kontaminiert werden. Stattdessen ist kann es vorteilhaft unschädlich sein, die Berührschutzscheibe 31 etwa mit einem Finger zu berühren.

Ab der in Fig. 7 gezeigten Stellung (entspricht dem Erstkontakt zwischen der Stirnfläche des Außenrohrs 3 und der zugeordneten Fläche der Berührschutzscheibe 31) wird die Berührschutzscheibe 31 vom Außenrohr 3 vorgetrieben und weitergeschoben. Dabei klappen die Biegesegmente derart um, dass es in vorteilhafter Weise zu keinem Zeitpunkt zu einer mechanischen Berührung zwischen Außenoberflächen der Berührschutzscheibe 31 und den Oberflächen des Konnektionsrohrs 5 und/oder den Oberflächen des Innenrohrs 1 und/oder den Trichterflächen des Außenrohrs 3 kommen kann.

Die Berührschutzscheibe 31 kann derart winkeltolerant gestaltet sein, dass ein Winkel in axialer oder Mittellinienrichtung zwischen der Verbindungseinrichtung 100 und der Konnektorbaugruppe 200 ungleich Null gestattet werden kann, ohne dabei die ebengeschilderte Funktion zu beeinträchtigen.

Der Aufnahmeraum 23 kann ausreichend geräumig ausgestaltet sein, um die Berührschutzscheibe 31 in jeder Stellung des Konnektionsrohrs 5 kraftarm aufnehmen zu können.

Aufgrund ihrer Verrastung und durch Reibung der umgebogenen Segmente kann ein sicherer Halt der Berührschutzscheibe 31 gegeben sein.

Im Gegensatz zu manchen herkömmlichen Berührschutzkappen oder Berührschutz-Abziehfolien behindert die Berührschutzscheibe 31 vorteilhaft die bekannten Verfahren der Gassterilisation nicht.

Die Berührschutzscheibe 31 ist in vorteilhafter Weise wie die übrige externe Funktionseinrichtung 400 aus Thermoplasten preisgünstig im Spritzguss herstellbar.

Nach ihrem Gebrauch kann die Stellung der nun nicht mehr an der Oberfläche der externen Funktionseinrichtung wahrnehmbare Berührschutzscheibe 31 dem Benutzer signalisieren, dass die externe Funktionseinrichtung 400 schon einmal gebraucht wurde.

Die Berührschutzscheibe 31 muss gegenüber einer herkömmlichen Verschlusskappe vorteilhaft nicht abgenommen werden und kann den Berührschutz der Konnektionsmündung lückenlos gewährleisten.

Sie kann alternativ auch als Hustschutzscheibe ausgeführt werden, wie in der EP 0 966 985 B1 beschrieben. Deren diesbezüglicher Offenbarungsgehalt wird durch Verweis an dieser Stelle aufgenommen. Damit kann in vorteilhafter Weise ein besonders hoher Schutz der Konnektionsanordnung oder der erfindungsgemäßen Verbindungseinrichtung 100 auch gegen Anhusten bzw. gegen Beaufschlagung mit Fremdkörpertröpfchen über die Luft gewährleistet werden.

Die Stirnseite des freien Endes 6 des Konnektionsrohrs 5 stellt eine potentielle Kontaminationszone dar. Wie in Fig. 10 gezeigt, ist am freien Ende 6 des Konnektionsrohrs 5 eine umlaufende Innen-Fase 8 bzw. Innen-Abrundung vorgesehen. Die Innen-Fase 8 berührt die Aufweitung 39 vorzugsweise nicht. Nur der Übergangsbereich der Innen-Fase 8 bzw. der Abrundung zum glatten Rohrabschnitt des Konnektionsrohrs 5 berührt die Aufweitung 39 des Innenrohrs 1, so dass eventuell in der Kontaminationszone befindliche Keime nicht in das Konnektionsrohr 5 hinein verschleppt werden können.

Diese Feinzentrierung kann eine hygienisch zulässige Erstberührung von relevanten Oberflächen des Konnektionsrohrs 5 und/oder des Innenrohrs 1 vorteilhaft erlauben oder begünstigen.

Auf diese Weise kann eine Kontaminationsverschleppung in die relevanten Mantelflächen des Konnektionsrohrs 5 vorteilhaft auch dann verhindert werden, wenn es dennoch zu einer Kontamination der Stirnfläche des Konnektionsrohrs 5 oder der Trichterfläche bzw. des Einführtrichters 87 des Außenrohrs 3 gekommen sein sollte.

Bei nur einmal zu verwendenden externen Funktionseinrichtungen 400 kann ein Wiederverwendungsschutz gewünscht sein. Ebenso kann gewünscht sein, dass nach der Entnahme der externen Funktionseinrichtung 400 aus der Behandlungsvorrichtung 300 keine Restflüssigkeiten aus der Konnektionsmündung des Konnektionsrohrs 5 der Konnektorbaugruppe 200 austreten können.

In einer weiteren bevorzugten Ausführungsform stellt die vorliegende Erfindung daher einen Wiederverwendungs- und/oder Auslaufschutz der Konnektorbaugruppe 200 bereit. Daneben kann wiederum vorteilhaft eine verbesserte Gassterilisierbarkeit gewährleistet werden.

Bei der erfindungsgemäßen Lösung kann nicht nur die erfolgte Erstverwendung der externen Funktionseinrichtung 400 angezeigt werden, sondern auch eine unzulässige Zweitverwendung verhindert werden.

Von Vorteil ist bei der erfindungsgemäßen Lösung ferner, wenn etwa vergessen wird, einen Originalitätsverschluss abzunehmen, und es dann - insbesondere bei automatischen Konnektionssystemen - zur Beschädigung der externen Funktionseinrichtung 400 und/oder der Behandlungsvorrichtung 300 kommen würde.

In einer weiteren bevorzugten Ausführungsform stellt die vorliegende Erfindung einen Wiederverwendungs- und/oder Auslaufschutz der Konnektorbaugruppe 200 bereit. Daneben kann wiederum vorteilhaft eine verbesserte Gassterilisierbarkeit gewährleistet werden.

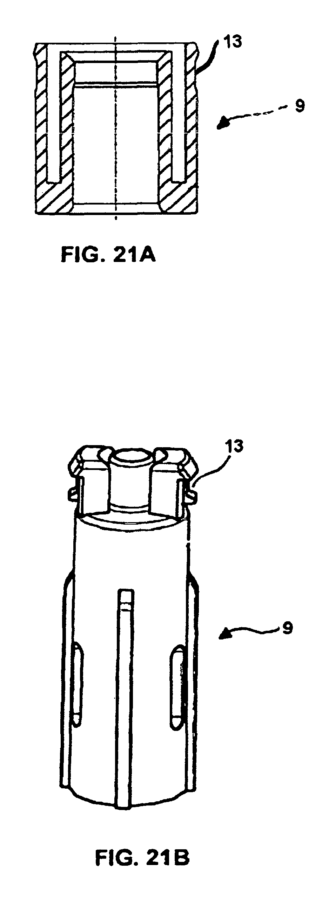

Dies wird erfindungsgemäß durch die Verschlusshülsen 9 erreicht, beispielsweise nach den Figuren 21A, 21B, 22A, 22B sowie 26.

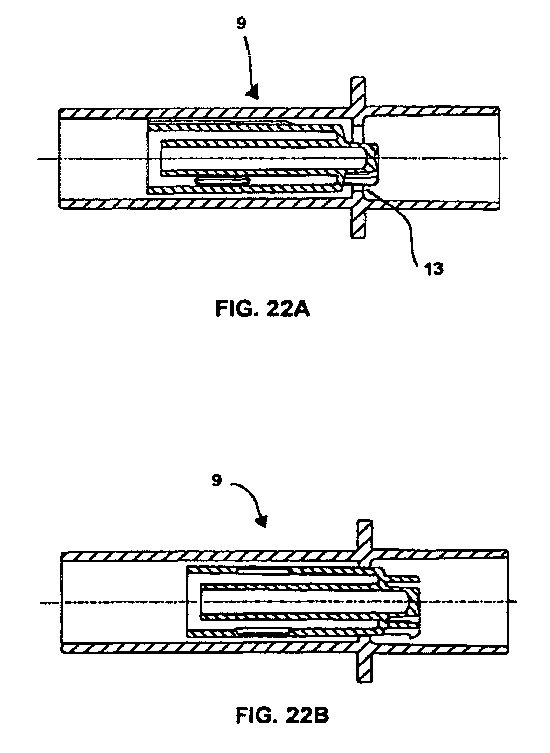

In den Fig. 22A und 22B is eine Verschlusshülse 9 mit integriertem Tropfschutz gezeigt. Der Tropfschutz wird dadurch erreicht, dass mehrere Rohrabschnitte konzentrisch ineinander liegend vorgesehen sind, so dass enge Ringspalte entstehen, die aufgrund der Kapillarwirkung Tropfen "aufsaugen" und somit ein Herauslaufen verhindern. Eine derart aufgebaute Verschlusshülse 9 kann in einer Vielzahl recht unterschiedlicher externer Funktionseinrichtungen (Disposables) verwendet werden, immer dort, wo nach dem Dekonnektieren das Herauslaufen von Flüssigkeit auftreten kann. Insbesondere bietet sich ein Einsatz in den Dialysatanschlüssen von Dialysatoren an.

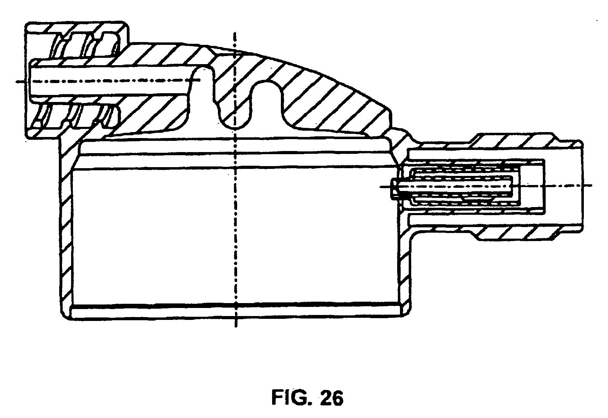

Fig. 26 zeigt eine Endkappe eines Dialysators mit eingesetzter Verschlusshülse 9. Es ist denkbar, die in den Fig. 22A und 22B gezeigte Verschlusshülse 9 als Nachrüstsatz für bereits am Markt verfügbare Dialysatoren einzusetzen. Die Verschlusshülse 9 mit integriertem Tropfschutz ist initial geöffnet, so dass eine Gassterilisation und/oder Dampfsterilisation durchgeführt werden kann. Sie ist nach Ende der Einmalverwendung der externen Funktionseinrichtung irreversibel verschlossen, wobei der Tropfschutz ein Herauslaufen von Flüssigkeitstropfen aus dem Stutzen verhindert.

Fig.22A zeigt die Durchströmungsstellung, Fig. 22B die Verschlussstellung.

Im Rahmen der Fertigung der externen Funktionseinrichtung 400 kann die Verschlusshülse 9 aus den gleichen preiswerten Thermoplasten wie die übrigen Bestandteile der externen Funktionseinrichtung 400 hergestellt sein. Sie kann durch die Rastanordnung 13 gehalten werden.

Die initiale Anordnung kann in vorteilhafter Weise besonders einfach und effektiv jede Form der Gassterilisation erlauben.

Durch die spannungsfreie Verrastung treten bei der Sterilisation - insbesondere auch bei Dampfsterilisationen - keine Schädigungen an der externen Funktionseinrichtung 400 auf.

Nach Ende der Fluidbehandlung kann die Verbindungseinrichtung 100 der Behandlungsvorrichtung 300 die beiden Konnektionssysteme (Verbindungseinrichtung 100 und Konnektorbaugruppe 200 bzw. externe Funktionseinrichtung 400 oder Behandlungsvorrichtung 300) noch weiter ineinander fahren, wodurch die Verschlusshülse 9 in die Verschlussstellung gelangt.

Dabei kann es vorteilhaft zu einer flüssigkeitsdichten und/oder unlösbaren Aufweitabdichtung des Konnektionsrohrs 5 der externen Funktionseinrichtung 400 zwischen den einander zugeordneten Topologien von Verschlusszapfen 17 zu Verschlusskragen 11 kommen.

Eine Wiederverwendung kann dadurch in vorteilhafter Weise sicher ausgeschlossen werden und kann im Rahmen der Inititaltests nach Einbau der externen Funktionseinrichtung 400 (z.B. Druckhaltetest) auch sicher erkannt werden.

Die Behandlungsvorrichtung 300 und/oder die externe Funktionseinrichtung 400 werden beim Versuch der Wiederverwendung vorteilhaft nicht beschädigt.

Beim Dekonnektieren der Verbindungseinrichtung 100 und der Konnektorbaugruppe 200 verbleibt immer noch ein Restraum im Konnektionsrohr 5 mit einer Restflüssigkeit, die nach Möglichkeit nicht in die Umgebung austreten soll.

Durch die eng angeordneten koaxialen Rohrstrukturen und/oder Durchtrittsbohrungen in Verbindung mit dem geringen Abstand der Stirnfläche des Innenrohrs 1 zur berührenden Stirnfläche der Verschlusshülse 9 kann vorteilhaft nur ein geringes Volumen an Restflüssigkeit verbleiben, welches zudem durch die engen Strukturen über den Kapillareffekt vorteilhaft am Abtropfen gehindert werden kann.

Diese Kapillar-Bauweise kann im Fall des bekannten Rinse- bzw. Online-Konnektors in das Konnektionsrohr 5 direkt integriert sein.

Eine Verschlusshülse kann in dieser Anwendung ganz eingespart werden. Die Zahl der Bestandteile des Konnektors verringert sich bei Gewährleistung der Gassterilisierbarkeit gegenüber herkömmlichen Einrichtungen auf nur zwei Teile.

Ein weitere vorteilhafte Anwendung des Wiederverwendungsschutzes und Auslaufschutzes kann sich bei den Dialysatanschlüssen der Dialysefilter gemäß Fig. 26 ergeben.

Bisher mussten Verschlusskappen i.d.R. mitgeliefert und manuell nach Gebrauch aufgesteckt werden, damit ein Auslaufschutz von Rest-Dialysierflüssigkeit nach Gebrauch gegeben war.

Die Verwendung der Verschlusskappen ist besonders unpraktisch, weil sie nach dem Abnehmen vorschriftsmäßig entsorgt werden müssen und bei Bedarf durch neue sterile Kappen ersetzt werden müssen.

Weiterhin ist es i.d.R. erforderlich, erst umständlich einen oben liegenden maschinenseitigen Dialysatanschluss abzunehmen, die obere Verschlusskappe manuell aufzusetzen, dann den Filter um 180 Grad zu wenden und die gleiche Prozedur am anderen Dialysatanschluss durchzuführen zu dem Zweck, das Auslaufen des Dialysators zu verhindern

Mit der erfindungsgemäßen Ausgestaltung des Innenrohrs 1 und der verrastet eingesteckten Verschlusshülse 9 (siehe z.B. Fig. 26) kann sich vorteilhaft ein Dialysefilter ergeben, welcher im Bezug auf die Hansen-Konnektorgeometrie und/oder im Bezug auf die Konnektionen im Rahmen der Filterherstellung voll kompatibel zu den üblichen Dialysefiltern sein kann. Es ist ein Einsatz in jedem beliebigen Dialysefilter am Markt denkbar.

Mögliche Mehrkosten bei der Herstellung können insbesondere unwesentlich sein.

Die Flüssigkeits-, Gas- und/oder Dampfbehandlung der Dialysefilter bei der Herstellung ist vorteilhaft ohne Einschränkungen weiterhin gegeben. Ein gemischter Einsatz von Filtern mit und ohne Verschlusshülse 9 kann durch entsprechende, auf die Hansen-Konnektorgeometrie bezogene Ausführung der Verbindungseinrichtung 100 gleichermaßen gewährleistet werden.

Die vorliegende Erfindung stellt somit eine optimierte Wiederverwendungssperre und/oder einen handlingoptimierten Auslaufschutz bereit.

Der eine der beiden Antriebe ist der Linearantrieb für die Haupt-Konnektionsbewegung, welcher nicht nur zwei Stellungen, sondern mindestens die drei Stellungen Grundposition, Konnektionsposition und Verschlussposition zulässt und bei entsprechender Konnektionsweise wie oben beschrieben auch weitere Stellungen vorsieht.

Es kommen bevorzugt Elektroantriebe in Verbindung mit absoluten Wegmeßsystemen und freier Programmierbarkeit der Verfahrprogramme zum Einsatz.

Der Einsatz einer Gewindespindel-Gewindemutter zur Umsetzung der Drehbewegung in eine Linearbewegung ist im Vergleich zu andern Umsetzungsmöglichkeiten besonders preiswert. Ihr Einsatz kann zudem den Vorteil aufweisen, bei hinreichend niedrig gewählter Gewindesteigung den Linearantrieb bei Funktionsausfall in der aktuellen Stellung zu haiten.

Der zweite Antriebskomplex betrifft den Antrieb der für das Aufsetzen bzw. Entfernen der Verschluss- bzw. Spülkappe 41 erforderlichen Bewegungen. Hier kann vorzugsweise und vorteilhaft ein kombinierter Schwenk- und/oder Hubantrieb mit elektromotorischer Ansteuerung zum Einsatz kommen. Auch andere Bewegungs- und Betätigungsarten wie etwa eine Schrägschieberbewegung oder ein Antrieb mittels Druckluftzylindern sind von der Erfindung umfasst.

Schließlich kann es vorteilhaft möglich sein, aus der Hubbewegung des linearen Hauptantriebs durch entsprechende Anschläge oder Steuerkurven auch die Bewegungen des Spülkappen-Schwenkantriebs 43 mechanisch abzuleiten. Diese Vorgehensweise ist ebenfalls versuchsweise erfolgreich umgesetzt worden.

Fig. 4 zeigt eine Radialabdichtung, welche in vorteilhafter Weise ganz auf elastomere Dichtringe (mit ihren Totraumproblemen) verzichten kann. Mindestens einer der beiden Dichtpartner (der Dichtkörper 59 des Außenrohrs 3 oder der Fluideinsatz 411 der Spülkappe 41) ist vorzugsweise einstückig aus einem zur Abdichtung wie zur Spülraumgestaltung geeigneten Werkstoff wie beispielsweise PTFE hergestellt.

Die zur Dichtung erforderliche elastische Nachgiebigkeit kann durch eine nachgiebige Gestaltung der Dichtzone und/oder durch eine zusätzliche Rückfederunterstützung, beispielsweise durch einen in die Abdichtrichtung wirkenden Elastomer-Ring und/oder durch entsprechend wirkende Federkonstruktionen (bekannt aus dem Bereich der Wellendichtringe), gewährleistet werden.

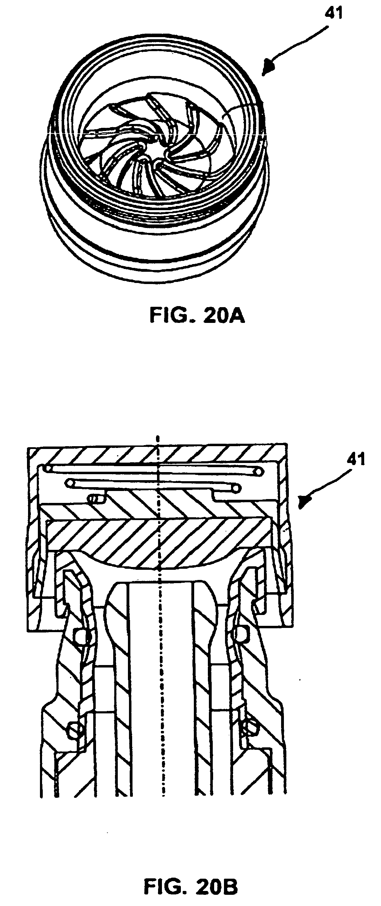

Eine weitere Variante der Spül- oder Verschlusskappenabdichtung gegen das Außenrohr 3 ist die in Figur 20B gezeigte axialdichtende Bauform: hier befindet sich ein Fluideinsatz 411 aus Elastomer oder aus PTFE, welcher axial dichtend auf der Stirnfläche des Außenrohrs 3 aufliegt. Auch hier gibt es vorzugsweise im Wesentlichen keine der vorgenannten typischen Toträume mehr. Als weiterer Vorteil kann eine besonders hohe Verträglichkeit gegen Abnützung hinzukommen, da sich eine solche Axialabdichtung selbsttätig axial und winkelig an den Dichtpartner anpassen kann.

Ein weiterer Vorteil dieser Verfahrensweise kann darin bestehen, dass der teilentleerte Innenraum der Verbindungseinrichtung 100 im Bereich der Aufweitung 39 des Innenrohrs 1 im Wesentlichen keinen zusammenhängenden Flüssigkeitsring beim Entfernen der Verschlusskappe bzw. Spülkappe 41 mehr aufweist, und dass auch der Raum vor der Stirnfläche des Innenrohrs 1 von Flüssigkeit teilentleert ist.

Der Montagekörper 413 der Spülkappe 41 in Fig. 4 ist mit einer Gewinde-, Steckrast- oder Bayonettverriegelung mit dem Spülkappen-Schwenkantrieb 43 verbunden. Außerdem ist die Dichtung des Montagekörpers 413 gegen das Chassis 47 der Behandlungsvorrichtung 300 bzw. gegen die Gummiplatte 49 vorzugsweise radial dichtend ausgeführt. Weiterhin weist der Montagekörper 413 auf der zum Anwender weisenden Stirnseite mindestens eine Einrichtung zur Übertragung der für die Demontage erforderlichen Kräfte und Bewegungen auf. Vorzugsweise handelt es sich dabei um eine Nut zur Demontage des Montagekörpers 413, welche vorteilhaft leicht und einfach bedienbar ist, wie beispielsweise mittels einer Münze.

Der Anwender kann nun die Spülkappe 41 vorteilhaft ohne weitere Zerlegung des Mechanismus herausnehmen und sowohl die Spülkappe 41 als auch den Stirnraum der Verbindungseinrichtung 100 beispielsweise einer Revision unterziehen. Nach Entfernung der Spülkappe 41 ist die Umgebung des Kopfkörpers 57 des Außenrohrs 3 zugänglich. Am Kopfkörper 57 des Außenrohrs 3 befinden sich außen mehrere Aus- oder Einnehmungen, an welchen ein Ringschlüssel-Werkzeug angreifen kann.

Somit können nicht nur Revisionen, sondern auch das Auswechseln der Spülkappe 41 wie auch des Dichtkörpers 59 des Außenrohrs 3(beides Verschleißteile) vorteilhaft ohne besonderen Aufwand vom Bedienpersonal der Behandlungsvorrichtung 300 ausgewechselt werden.

Die erfindungsgemäße Verbindungseinrichtung 100 nach Figur 3 und 4 beinhaltet in vorteilhafter Weise einen vollständigen Berühr- und/oder Hustschutz aller hygienerelevanten Bereiche. Die Spülkappe 41 ist in der Grundposition der Verbindungseinrichtung 100 gegen das Chassis 47 der Behandlungsvorrichtung 300 bzw. gegen die Gummiplatte 49 flüssigkeitsdicht gelagert.

Der erste Vorgang der Aufrüstung der Behandlungsvorrichtung 300 mit der externen Funktionseinrichtung 400 kann die Fixierung der externen Funktionseinrichtung 400 vor der Spülkappe 41 in Grundposition beinhalten. Somit kann in vorteilhafter Weise jede Berührung der hygienisch relevanten Bereiche ausgeschlossen werden. Unzugänglich für Berührungen und für Flüssigkeitstropfen aus der Umgebung wird im weiteren Prozess die Spülkappe 41 hinter der dichten Abdeckung durch die externe Funktionseinrichtung 400 von der Verbindungseinrichtung 100 abgezogen und in einen weiteren unzugänglichen Raum hinter der Front der Behandlungsvorrichtung 300 geschwenkt.

Für alle weiteren Vorgänge beim Gebrauch des Fluid-Konnektions-Systems bis einschließlich der Rückkehr der Spülkappe 41 in die Grundposition (Standby-Stellung bzw. Stellung für die Desinfektion und Spülvorgänge) bleibt die externe Funktionseinrichtung 400 bevorzugt in dieser Abdeck-Position.

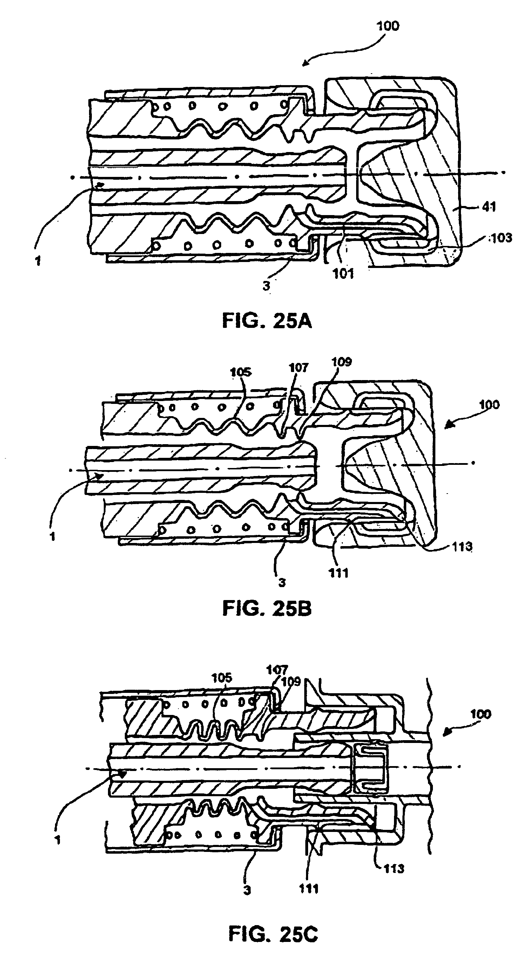

Die Fig. 25A bis 25C zeigen eine besonders optimierte Bauform und Bewegungsweise der erfindungsgemäßen Verbindungseinrichtung 100. Diese Anordnung kann insbesondere bei horizontaler Konnektionsanordnung weiter gesteigerte Ansprüche im Bezug auf geringe Verluste von Restflüssigkeit und/oder im Bezug auf eine Sicherstellung der Keimfreiheit erfüllen.

Fig. 20A zeigt einen Fluideinsatz 411 der Spülkappe 41 mit besonders optimierter Strömungsraumgestaltung. Durch eine im Wesentlichen kegelförmige Konturgebung in Verbindung mit spiralförmigen Umlenknuten wird die aus dem Innenrohr 1 beim Desinfizieren und/oder Spülen austretende Strömung vorzugsweise verlustarm zu den seitlichen Dichtbereichen zwischen Außenrohr 3 und Spülkappe 41 umgelenkt und dabei in eine Rotationsströmung versetzt.

Insgesamt wurde der Strömungsraum durch die optimierte Formgebung besonders eng gestaltet, um insgesamt hohe Strömungsgeschwindigkeiten und einen guten Fluidaustausch gewährleisten zu können.

Die Fig. 25A-C zeigen für die weiter optimierte Bauform die Komponenten Innenrohr 1, Außenrohr 3 und Spülkappe 41. Das Außenrohr 3 ist nun relativ zum Innenrohr 1 axial beweglich ausgestaltet.

Um radial dichtende Verschiebe-Dichtanordnungen mit ihren Abnützungs- und/oder Totraumproblemen zu vermeiden, ist diese Verschieblichkeit bevorzugt durch die gezeigte Faltenbalganordnung 105 aus einem Kunststoff wie PTFE realisiert.

An geodätisch tiefster Stelle ist im Außenrohr 3 ein Kanal 101 angeordnet, welcher in Rückströmrichtung zwischen zwei inneren Ringwülsten 107 und 109 mündet, welche durch die Verschieblichkeit des Innenrohrs 1 zum Außenrohr 3 in Verbindung mit entsprechenden Durchmesserverhältnissen relativ zum Dichtkopf oder Dichtkörper 59 eine schaltbare Strömungsdrossel bilden können.

Mindestens ein weiterer Kanal 103 befindet sich ebenfalls bevorzugt an tiefster Stelle in der Spülkappe 41. Das Außenrohr 3 weist bei der Konnektion mit der Spülkappe 41 zwei äußere Ringwülste 111 und 113 auf.

Figur 25A zeigt die Stellungen dieser Komponenten bei Desinfektion, Spülung und Bereitschaft. Die Spülkappe 41 ist maximal tief in das Außenrohr 3 eingeschoben. Der äußere Ringwulst 111 dient als finale Abdichtung zur Umgebung. Der weitere äußere Ringwulst 113 dient als Abstreifer für mögliche Verkrustungen im Bereich des Ringwulsts 111 und bildet in dieser Stelle eine Strömungsumlenkung zur Desinfektion und Spülung.

Das Innenrohr 1 ist in dieser Stellung so weit zurückgezogen, dass der Außendurchmesser des Dichtkörpers bzw. Dichtkopfes eine Strömungsdrossel gegen den weiteren inneren Ringwulst 109 bildet.

Die Strömung erfolgt zum größten Teil durch die Spülkappe 41, durch den Kanal 103 in der Spülkappe 41 in den Ringbereich zwischen den äußeren Ringwülsten 111, 113 in den Kanal 101 im Außenrohr 3 und von dort in den Abströmraum hinter den Dichtkörper bzw. Dichtkopf. Dabei wird der mündungsseitige Bereich des Außenrohrs 3 vorteilhaft besonders intensiv umspült und dabei den weiteren äußeren Ringwulst 113 von beiden Seiten gereinigt, während der äußere Ringwulst 111 an ihrer Seite zum Ringbereich gespült wird.

Der geringere Teil der Spülfluide findet den Weg durch die Drosselstelle am Dichtkopf. In der Bereitschaft bis zur nächsten Entfernung der Spülkappe 41 bleibt das Außenrohr 3 in einem besonders großen Bereich bis hin zum äußeren Ringwulst 111 vorteilhaft desinfiziert, gut gespült und/oder frei von möglichen Ablagerungen.

Figur 25B zeigt die Stellungen der Komponenten bei den Vorgängen der Entleerung und zu Beginn des nachfolgenden Konnektionsvorgangs mit dem Konnektionsrohr 5 der Konnektorbaugruppe 200.

Nach Abschluss der Desinfektions- und/oder Spülprozesse bewegt sich das Innenrohr 1 noch ein Stück weiter weg von der Stirnfläche des Außenrohrs 3. Dadurch kommt es zwischen dem weiteren inneren Ringwulst 109 und dem Außendurchmesser des Dichtkopfes zu einer annähernden Abdichtung.

Das Lumen des Außenrohrs 3 wird nun direkt oder über eine Fördereinrichtung an das Abwassernetz der Behandlungsvorrichtung 300 geschaltet. Das Innenrohr 1 wird direkt oder über eine Fördereinrichtung mit einem Gasanschluss verbunden. Durch Erzeugen einer Druckdifferenz bzw. durch das Aktivieren einer Fördereinrichtung wird die Flüssigkeit in Richtung des Abwassernetzes abgeführt.

In den dargestellten Passagebereichen im Bereich der Spitze des Innenrohrs 1 und des Außenrohrs 3 und der hierzu besonders angepasst gestalteten Spülkappe 41 (kurz Mündungsbereich) bleibt nur ein besonders geringes Volumen an Flüssigkeit, welches zur Trockenlegung der konnektionsrelevanten Konnektorbereiche entfernt werden muss. Aufgrund der engen Strömungsräume kann dieses Flüssigkeitsvolumen besonders effektiv in den geodätisch tiefer liegenden Faltenbalgbereich bzw. die Faltenbalg-Anordnung 101 des Ringlumens verschoben werden, von wo es bei horizontaler Konnektionsanordnung nicht mehr zurück in den Mündungsbereich gelangen kann.

Die relevanten Räume im Mündungsbereich der Verbindungseinrichtung 100 und der Spülkappe 41 werden derart vollständig von Flüssigkeit befreit, dass nur mehr ein kleiner Film von Restflüssigkeit an den Oberflächen verbleibt, welche beim nachfolgenden Abzug der Spülkappe 41 zu keinem Austreten von Restflüssigkeit in die Umgebung mehr führen kann.

Zu diesem Effekt trägt auch bei, dass die strömungsbeaufschlagten Oberflächen bevorzugt hydrophil beschichtet sind. Auf diese Weise können sich nur festhaftende Flüssigkeitsfilme und keine leicht beweglichen großen Flüssigkeitstropfen an den Oberflächen bilden.

Das Innenrohr 1 ist beim nachfolgenden Abziehen der Spülkappe 41 besonders weit von der Stirnfläche des Außenrohrs 3 und der Spülkappe 41 entfernt.

Das Innenrohr 1 ist beim nachfolgenden Abziehen der Spülkappe 41 besonders weit von der Stirnfläche des Außenrohrs 3 und der Spülkappe 41 entfernt. Darüber hinaus ist der gesamte bei den nachfolgenden Vorgängen relevante Mündungsbereich nahezu vollständig von Flüssigkeit bzw. von bewegungsfähiger Restflüssigkeit befreit.

Infolgedessen besteht für das weit zurückstehende und für die anschließende sterile Konnektion besonders relevante Innenrohr 1 ab dem Zeitpunkt unmittelbar vor Abzug der Spülkappe 41 bis zum Abschluss der sich anschließenden Konnektionsvorgänge ein besonders effektiver Schutz sowohl gegen Berührungen als auch gegen mögliche Kontaminationen mit unsterilen oder verschmutzenden Substanzen, insbesondere mit Flüssigkeitstropfen, welche während der nachfolgenden Spülkappen- und Konnektionsbewegungen in den dann gegenüber der unsterilen Umgebung offenen Mündungsbereich der internen Konnektorbaugruppe 200 gelangen können.

Zu Beginn der Konnektion mit der Konnektorbaugruppe 200 ist das Innenrohr besonders weit von der unsterilen Umgebung der Behandlungsvorrichtung 300 entfernt.

Fig. 25C zeigt die optimierte Bauform in der Stellung bei abgeschlossener Konnektion bis zum finalen Stellungswechsel in die Verschlussposition der Verschlusshülse 9.

Das Innenrohr 1 wird während der Konnektion zunehmend nach vorne in Richtung Stirnfläche des Außenrohrs 3 verschoben. Gleichzeitig dichten beide äußeren Ringwülste 111 und 113 gegen die zylindrische Wandung des Aufnahmeraums 23 ab.

Auf diese Weise werden die Funktionalitäten des Außenlumens 37 in Verbindung mit der redundanten Dichtung und/oder in Verbindung mit der Dichtheitsüberprüfung aufrechterhalten. Beim Erreichen der Konnektionsstellung und beim Wechsel in die Verschlussposition kann das Innenrohr 1 zunehmend weiter in Richtung Stirnfläche des Außenrohrs 3 verschoben werden und damit weiter als bei einer starren Anordnung des Innenrohrs 1 relativ zum Außenrohr 3. Auf diese Weise lässt sich eine ausreichend große Axialtoleranz mit geringerer Baulänge des Konnektionsrohrs 5 und des Aufnahmeraums 23 verwirklichen.

Jeder Ringwulst kann vorzugsweise als eingelegter Dichtring aus mit PTFE-überzogenem Elastomerwerkstoff gefertigt sein.

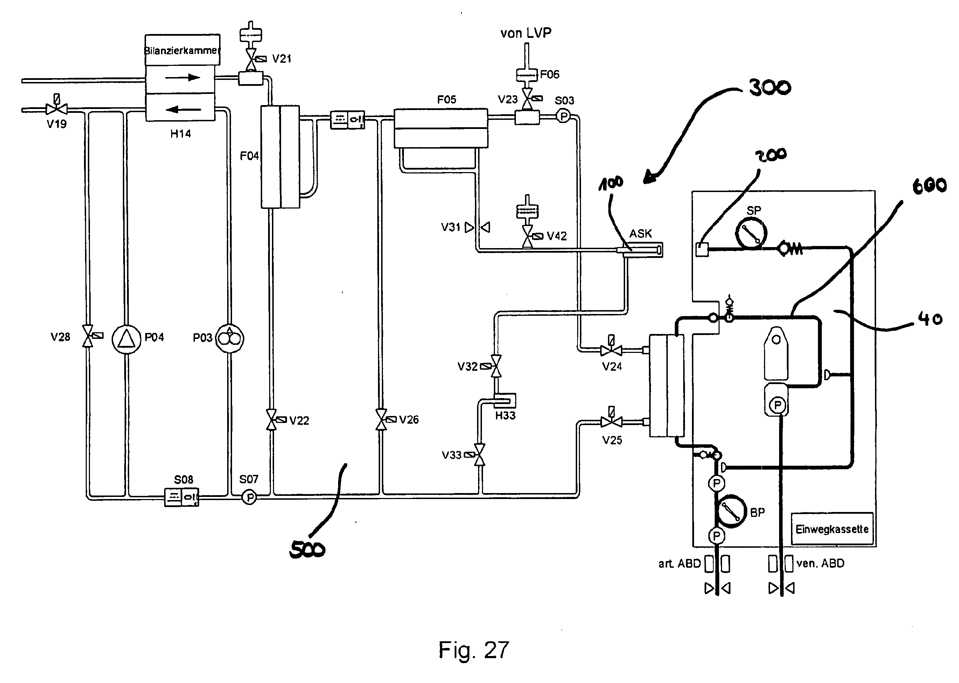

**Fig. 27** zeigt schematisch die Lage der erfindungsgemäßen Verbindungseinrichtung 100 in einem Verfahrensfließbild einer Dialysemaschine als Behandlungsvorrichtung 300 mit Dialysatkreislauf 500 und extrakorporalem Blutkreislauf 600. Der extrakorporale Blutkreislauf 600 weist eine externe Funktionseinrichtung 400 auf, die beispielhaft als Blutbehandlungskassette gezeigt ist. Eine derartige Blutbehandlungskassette ist beispielsweise in der von der Anmelderin der vorliegenden Anmeldung beim DPMA am 23. April 2009 eingereichten Anmeldung 10 2009 018 664 A1 bzw. in der am 10. Juni eingereichten Anmeldung 10 2009 024 468 A1 mit jeweils dem Titel *"Externe Funktionseinrichtung, Blutbehandlungsvorrichtung zum Aufnehmen einer erfindungsgemäßen externen Funktionseinrichtung, sowie Verfahren"* beschrieben, auf deren diesbezügliche Offenbarungen hiermit vollinhaltlich Bezug genommen wird.

Die externe Funktionseinrichtung 400 weist als integralen Bestandteil die Konnektorbaugruppe 200 auf. Die Verbindungseinrichtung 100 und die Konnektorbaugruppe 200 bilden die Substituatverbindung von der Dialysemaschine zur Blutbehandlungskassette. In Fig. 27 sind die Verbindungseinrichtung 100 und die Konnektorbaugruppe 200 im nicht konnektierten Zustand dargestellt.

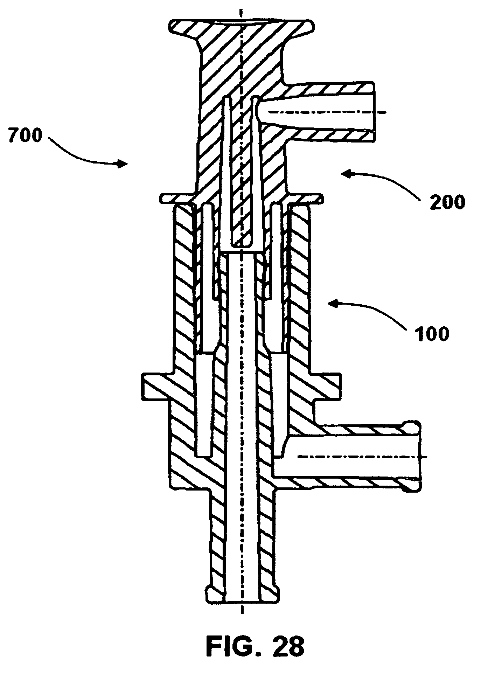

**Fig. 28** zeigt einen Rinse-Port 700 im Längsschnitt.

Der Rinse-Port 700 weist eine Ausführung beider Konnektorbaugruppen bzw. Konnektions-Halbanordnungen, d.h. sowohl die Konnektorbaugruppe 200 als auch die Verbindungseinrichtung 100, auf Eine derartige Anordnung kommt insbesondere bei Anwendungen zum Einsatz, bei denen eine Konnektion manuell zustande kommt und/oder eine Vorzentrierung durch die Konnektorbaugruppen direkt zustande kommt und/oder auf zusätzliche Einrichtungen des lateralen Toleranzausgleichs verzichtet wird und/oder die interne Konnektorbaugruppe wie die externe Konnektorbaugruppe ebenfalls als Disposable ausgeführt wird und/oder auf eine Verschlusshülse verzichtet werden kann, da der kapillare Tropfschutz bereits einstückig in das Konnektionsrohr der externen Konnektorbaugruppe integriert ist und wegen der hier nur geringen Drücke, unter welchen mögliche Restflüssigkeiten an der externen Konnektorbaugruppe anstehen, eine Verschlusshülse als Tropfschutz nicht erforderlich ist.

Fig. 28 verdeutlicht auch, dass beide Konnektorbaugruppen jeweils für sich genommen über einen effektiven klassischen Berührschutz durch zurückgesetzte konnektionsrelevante Innenkonnektoren (Innenrohr und Konnektionsrohr) verfügen.

## Patentansprüche

1. Verbindungseinrichtung (100) zur Fluidverbindung wenigstens eines ersten mit einem zweiten fluidführenden medizintechnischen System mit
- wenigstens einem ersten Rohrstück des ersten Systems, welches vorgesehen ist, mit einem zweiten Rohrstück des zweiten Systems verbunden zu werden; wobei
- das erste Rohrstück wenigstens ein Innenrohr (1) und wenigstens ein Außenrohr (3) aufweist, mit einem Raum (37) zwischen dem Innenrohr (1) und dem Außenrohr (3); wobei
- das zweite Rohrstück ein Konnektionsrohr (5) ist; und wobei
- der Raum (37) zwischen dem Innenrohr (1) und dem Außenrohr (3) geeignet ist zur Aufnahme wenigstens eines Abschnitts des Konnektionsrohrs (5); mit
- einer Aufweitung (39) zum Verbinden des ersten Rohrstücks, mit dem zweiten Rohrstück;
**dadurch gekennzeichnet, dass**
- die Aufweitung (39) am Innenrohr (1) einstückig hergestellt vorliegt.

2. Verbindungseinrichtung (100) nach Anspruch 1, wobei das erste Rohrstück ein Innenrohr (1) und das zweite Rohrstück ein Konnektionsrohr (5) ist.

3. Verbindungseinrichtung (100) nach einem der vorangegangenen Ansprüche, welche eine Verschlusseinrichtung (41) aufweist zum Kurzschließen oder Verbinden des Innenlumens des Innenrohrs (1) mit dem Raum zwischen Innenrohr (1) und Außenrohr (3) in Fluidverbindung.

4. Verbindungseinrichtung (100) nach einem der vorangegangenen Ansprüche, wobei die Aufweitung (39) eine kugelförmige und/oder sphärische und/oder gekrümmte und/oder bogenförmige und/oder konvexe Außenoberfläche, insbesondere in einem Bereich des maximalen Querschnitts oder Durchmessers, vorzugsweise in Strömungsrichtung, aufweist.

5. Verbindungseinrichtung (100) nach einem der vorangegangenen Ansprüche, wobei die Aufweitung (39) ausgestaltet ist, im gebrauchsfertigen Verbindungszustand der Verbindungseinrichtung (100) eine Fluidabdichtung zwischen dem erstem Rohrstück und dem zweitem Rohrstück, insbesondere zwischen Innenrohr (1) und Konnektionsrohr (5), zu bewirken.

6. Verbindungseinrichtung (100) nach einem der vorangegangenen Ansprüche, wobei die Aufweitung (39) derart ausgestaltet ist, dass sie beim Verbinden des ersten Rohrstücks mit dem zweiten Rohrstück entlang eines Verschiebewegs im Rohrstücks, in welches sie eingeführt wird, unter Beibehaltung des durch sie bewirkten Abdichtzustands bewegbar ist, und/oder derart ausgestaltet ist, dass sie beim Verbinden des ersten Rohrstücks mit dem zweiten Rohrstück oder nach Abschluss des Verbindens um einen Winkel bezogen zur Strömungsrichtung neigbar ist, unter Beibehaltung des durch sie bewirkten Abdichtzustands.

7. Verbindungseinrichtung (100) nach einem der vorangegangenen Ansprüche, welche eine weitere Abdichteinrichtung (91) aufweist.

8. Verbindungseinrichtung (100) nach einem der vorangegangenen Ansprüche, mit einer Schwenklagerung (455) zur Ausrichtbarkeit des ersten Rohrstücks und/oder des zweiten Rohrstücks zueinander.

9. Verbindungseinrichtung (100) nach einem der vorangegangenen Ansprüche, mit einer Vorzentriereinrichtung zur horizontalen und/oder lateralen Ausrichtbarkeit des ersten Rohrstücks und/oder des zweiten Rohrstücks.

10. Verbindungseinrichtung (100) nach Anspruch 9, wobei die Vorzentriereinrichtung wenigstens zwei federnd auslenkbare Zungen aufweist.

11. Verbindungseinrichtung (100) nach einem der vorangegangenen Ansprüche, mit einer Einrichtung, welche beim Konnektieren derart mechanisch verändert und/oder bewegt wird, dass die erfolgte Konnektion nach Dekonnektion an der Einrichtung erkennbar bleibt.

12. Verfahren zum Konnektieren von wenigstens zwei fluidführenden medizintechnischen Systemen, **gekennzeichnet durch** die Verwendung wenigstens einer Verbindungseinrichtung (100) nach einem der Ansprüche 1 bis 11.

13. Verfahren nach Anspruch 12, mit dem Schritt:
- Aufweiten des Innenlumens (7) des zweiten Rohrstücks, insbesondere des Konnektionsrohrs (5), mittels der Aufweitung (39) des ersten Rohrstücks, insbesondere des Innenrohrs (1), zum Erzielen einer fluiddichten Verbindung.

14. Externe, medizintechnische Funktionseinrichtung (400), ausgestaltet als eine Blutkassette für die extrakorporale Blutbehandlung, wenn mit einer Verbindungseinrichtung (100) gemäß einem der Ansprüche 1 bis 11 verbunden.

15. Medizintechnische Vorrichtung (300) mit
- wenigstens einer Verbindungseinrichtung (100) gemäß einem der Ansprüche 1 bis 11 und/oder
- wenigstens einer externen Funktionseinrichtung (400) gemäß Anspruch 14 und/oder
- einer Steuereinrichtung, konfiguriert zum Ausführen eines Verfahrens gemäß einem der Ansprüche 12 oder 13; wobei
die Vorrichtung (300) eine Blutbehandlungsvorrichtung ist.

## Claims

1. A connection means (100) for fluid connection of at least one first to one second fluid-conducting medical-technical system, comprising
- at least one first pipe section of the first system, which is intended for being connected to a second pipe section of the second system, wherein
- the first pipe section comprises at least one inner pipe (1) and at least one outer pipe (3), with a space (37) between the inner pipe (1) and the outer pipe (3); wherein
- the second pipe section is a connection pipe (5); and wherein
- the space (37) between the inner pipe (1) and the outer pipe (3) is suited for receiving at least a portion of the connection pipe (5), with
- a widening (39) for connecting the first pipe section to the second pipe section;
**characterized in that**
- the widening (39) is integrally manufactured with the inner pipe (1).

2. The connection means (100) according to claim 1, wherein the first pipe section is an inner pipe (1) and the second pipe section is a connection pipe (5).

3. The connection means (100) according to any one of the preceding claims, which comprises a closure means (41) for short-circuiting or connecting the inner lumen of the inner pipe (1) with the space between inner pipe (1) and outer pipe (3) in fluid communication.

4. The connection means (100) according to any one of the preceding claims, wherein the widening (39) has a spherical and/or globular and/or curved and/or arc-shaped and/or convex outer surface, in particular in a region of the maximum cross-section or diameter, preferably in a direction of flow.

5. The connection means (100) according to any one of the preceding claims, wherein the widening (39) is configured to effect a fluidic seal between the first pipe section and the second pipe section, in particular between inner pipe (1) and connection pipe (5), in the ready-to-use, connected condition of the connection means (100).

6. The connection means (100) according to any one of the preceding claims, wherein the widening (39) is configured such that it may be moved, in the process of connecting the first pipe section to the second pipe section, along a displacement path in the pipe section into which it is being introduced, while maintaining the sealing condition brought about by it, and/or is configured such that it may be tilted, in the process of connecting the first pipe section to the second pipe section or following completion of connecting, by an angle relative to the direction of flow, while maintaining the sealing condition brought about by it.

7. The connection means (100) according to any one of the preceding claims, which comprises a further sealing means (91).

8. The connection means (100) according to any one of the preceding claims, comprising a pivotal mount (455) for a capability of aligning the first pipe section and/or the second pipe section relative to each other.

9. The connection means (100) according to any one of the preceding claims, comprising a pre-centering means for a capability of horizontal and/or lateral alignment of the first pipe section and/or of the second pipe section.

10. The connection means (100) according to claim 9, wherein the pre-centering means comprises at least two springily deflectable tongues.

11. The connection means (100) according to any one of the preceding claims, comprising a means that is mechanically altered and/or moved in the process of connecting such that the completed connection continues to be apparent on the means following disconnection.

12. A method for connecting at least two fluid-conducting medical-technical systems, **characterized by** the use of at least one connection means (100) according to any one of claims 1 to 11.

13. The method according to claim 12, including the step of:
- expanding the inner lumen (7) of the second pipe section, in particular of the connection pipe (5), by means of the widening (39) of the first pipe section, in particular of the inner pipe (1), for obtaining a fluid-tight connection.

14. An external, medical-technical functional means (400) configured as a blood cassette for the extracorporeal blood treatment, when connected to a connection means (100) according to any one of claims 1 to 11.

15. A medical-technical apparatus (300) comprising:
- at least one connection means (100) according to any one of claims 1 to 11 and/or
- at least one external functional means (400) according to claim 14 and/or
- a control means configured for performing a method according to any one of claims 12 or 13; wherein.
the apparatus (300) is a blood treatment apparatus.

## Revendications

1. Un moyen de connexion (100) pour connexion fluidique entre au moins un premier et un second système médicotechniques conducteurs de fluide avec:
- au moins une première section de tube du premier système, laquelle est prévue pour être connectée à une seconde section de tube du second système ; où
- la première section de tube comprend au moins un tube intérieur (1) et au moins un tube extérieur (3) avec un espace (37) entre le tube intérieur (1) et le tube extérieur (3); où
- la seconde section de tube est un tube de raccordement (5); et où
- l'espace (37) entre le tube intérieur (1) et le tube extérieur (3) est adapté pour recevoir au moins une partie du tube de raccordement (5); avec
- un élargissement (39) pour connecter la première section de tube avec la seconde section de tube;
**caractérisé en ce que**
- l'élargissement (39) est réalisé d'une pièce avec le tube intérieur (1).

2. Le moyen de connexion (100) selon la revendication 1, où la première section de tube est un tube intérieur (1) et où la seconde section de tube est un tube de raccordement (5).

3. Le moyen de connexion (100) selon l'une quelconque des revendications précédentes, comprenant un moyen de fermeture (41) pour court-circuiter ou connecter le lumen interne du tube intérieur (1) avec l'espace entre le tube intérieur (1) et le tube extérieur (3) en connexion fluidique.

4. Le moyen de connexion (100) selon l'une quelconque des revendications précédentes, où l'élargissement (39) présente une surface extérieure de forme globulaire et/ou sphérique et/ou courbée et/ou en forme d'arc et/ou convexe, en particulier au voisinage d'une section transversale maximale ou de plus grand diamètre, de préférence dans le sens d'écoulement.

5. Le moyen de connexion (100) selon l'une quelconque des revendications précédentes, où l'élargissement (39) est constitué de façon à réaliser un joint étanche aux fluides entre la première section de tube et la seconde section de tube, en particulier entre le tube intérieur (1) et le tube de raccordement (5) dans un état de connexion lors de l'usage du moyen de connexion (100).

6. Le moyen de connexion (100) selon l'une quelconque des revendications précédentes, où l'élargissement (39) est constitué de façon à être déplaçable le long d'une course de déplacement à l'intérieur de la section de tube dans laquelle il est introduit lors de l'établissement de la connexion de la première section de tube avec la seconde section de tube tout en maintenant l'état d'étanchéité réalisé par celui-ci et/ou où l'élargissement (39) est constitué de façon à être inclinable d'un certain angle par rapport au sens d'écoulement lors de l'établissement de la connexion de la première section de tube avec la seconde section de tube, ou à l'issue de la connexion, tout en maintenant l'état d'étanchéité réalisé par celui-ci.

7. Le moyen de connexion (100) selon l'une quelconque des revendications précédentes, présentant un moyen d'étanchéité (91) supplémentaire.

8. Le moyen de connexion (100) selon l'une quelconque des revendications précédentes avec un support pivotant (455) permettant l'orientation de la première section de tube et/ou de la seconde section de tube l'une par rapport à l'autre.

9. Le moyen de connexion (100) selon l'une quelconque des revendications précédentes avec un moyen de pré-centrage permettant l'orientation horizontale et/ou latérale de la première section de tube et/ou de la seconde section de tube.

10. Le moyen de connexion (100) selon la revendication 9, où le moyen de pré-centrage comprend au moins deux languettes fléchissables élastiquement.

11. Le moyen de connexion (100) selon l'une quelconque des revendications précédentes avec un dispositif pouvant être modifié mécaniquement et/ou pouvant être déplacé lors de la connexion de telle sorte que la connexion effectuée reste reconnaissable sur le dispositif après déconnexion.

12. Procédé pour connecter au moins deux systèmes médicotechniques conducteurs de fluide, **caractérisé par** l'usage d'au moins un moyen de connexion (100) selon l'une quelconque des revendications 1 à 11.

13. Procédé selon la revendication 12, comprenant l'étape:
- élargir le lumen interne (7) de la seconde section de tube, en particulier du tube de raccordement (5), au moyen de l'élargissement (39) de la première section de tube, en particulier du tube intérieur (1), afin d'obtenir une connexion étanche.

14. Dispositif fonctionnel externe médicotechnique (400), constitué en tant que cassette à sang pour le traitement extracorporel du sang lorsqu'il est connecté avec un moyen de connexion (100) selon l'une quelconque des revendications 1 à 11.

15. Appareil médicotechnique (300) avec
- au moins un moyen de connexion (100) selon l'une quelconque des revendications 1 à 11 et/ou
- au moins un dispositif fonctionnel externe (400) selon la revendication 14 et/ou
- un dispositif de commande configuré pour exécuter un procédé selon l'une quelconque des revendications 12 ou 13; où
l'appareil (300) es un appareil de traitement du sang.
